# EUROPEAN PATENT SPECIFICATION

(11) **EP 1 963 357 B1**
(45) Date of publication and mention of the grant of the patent: **09.12.2009**
(21) Application number: 06820951.9
(22) Date of filing: 22.11.2006
(51) Int. Cl.: C07K 5/087, C07K 7/64, C07K 5/083, C07K 5/097

(54) **COMPOUNDS FOR THE INHIBITION OF APOPTOSIS**
VERBINDUNGEN ZUR INHIBIERUNG VON APOPTOSE
COMPOSES POUR L INHIBITION DE L APOPTOSE

(30) Priority: 23.11.2005 ES 200502890
(43) Date of publication of application: 03.09.2008
(73) Proprietor: Laboratorios SALVAT, S.A., 08950 Esplugues de Llobregat, Barcelona (ES); CONSEJO SUPERIOR DE INVESTIGACIONES CIENTIFICAS, 28006 Madrid (ES)
(72) Inventor: PEREZ PAYÁ, Enrique, E-46020 Valencia (ES); VICENT DOCON, Maria, Jesús, Vila-real, (Castellon) (ES); ORZAEZ CALATAYUD, Mar, E-46006 Valencia (ES); MONDRAGON MARTINEZ, Laura, E-12480 Soneja (Castellon) (ES); MESSEGUER PAYPOCH, Angél, E-08025 Barcelona (ES); MOURE FERNANDEZ, Alejadra, Barcelona (ES); SANCLIMENS PEREZ DE ROZAS, Gloria, Barcelona (ES); MALET ENGRA, Gema, E-46920 Mislata-Valencia (ES)
(74) Representative: Carvajal y Urquijo, Isabel
(86) International application number: PCT/IB2006/003312
(87) International publication number: WO 2007/060524

(56) References cited:
- WO-A-99/46248
- LURA R ET AL: "Role of peptide conformation in the rate and mechanism of deamidation of asparaginyl residues" BIOCHEMISTRY 1988 UNITED STATES, vol. 27, no. 20, 1988, pages 7671-7677, XP002426034 ISSN: 0006-2960
- ASHWELL S: "Caspases: Recent advances in small molecule inhibitors" EXPERT OPINION ON THERAPEUTIC PATENTS, ASHLEY PUBLICATIONS, GB, vol. 11, no. 10, 2001, pages 1593-1603, XP002315131 ISSN: 1354-3776

## Description

The present invention relates to compounds acting as apoptosis inhibitors, as well as to processes for their preparation, to pharmaceutical compositions containing them and their use in medicine.

### BACKGROUND ART

Apoptosis is an interesting biological process because of its importance in a wide variety of biological systems, including normal cell turnover, the immune system and embryonic development and its association with different diseases. inappropriate apoptosis is involved in many human pathologies, including neurodegenerative diseases such as Alzheimer's and Huntington's, ischaemia, autoimmune disorders and several forms of cancer (Reed J.C., Trends Mol. Med. 2001, 7: 314-319).

Diverse apoptotic stimuli, including activation of cell surface death receptors, anticancer agents, irradiation, lack of survival factors, and ischaemia (reviewed in Strasser A. et al., Annu. Rev. Biochem. 2000, 69: 217-245) induce signalling cascades that all activate a family of cysteine aspartyl proteases called caspases. These proteases execute the apoptotic process.

Some apoptotic signals activate the mitochondria-mediated or intrinsic pathway that utilises caspase-9 as its initiator. The formation of the macromolecular complex named apoptosome is a key event in this pathway.

The apoptosome is a holoenzyme multiprotein complex formed by cytochrome c-activated Apaf-1 (apoptotic protease-activating factor), dATP and procaspase- 9 (Li P. et al., Cell 1997, 91: 479-489; Acehan D et al., Mol. Cell 2002, 9: 423-432; Rodriguez J. et al., Genes Dev. 1999, 13: 3179-3184; Srinivasula S.M. et al., Mol. Cell 1998, 1: 949-957). In this macromolecular complex, apoptosome associated caspase-9 is activated and then, in turn, activate effector caspases. TO identify molecules that could ameliorate disease-associated apoptosis, drug discovery efforts have initially targeted caspase activity, and thus sought specific enzymatic activators or inhibitors (Scott C.W. et al., J. Pharmacol. Exp. Ther. 2003, 304: 433-440; Garcia-Calvo M, J. Biol. Chem. 1998, 273: 32608-32613), rather than modulators in the upstream activation cascade/pathway.

Nevertheless, protein-protein interactions upstream of caspase activation can be also relevant points of intervention for the development of modulators of apoptosis pathways. In particular, recent data propose the formation of the apoptosome as an interesting target for the development of apoptotic modulators.

Therefore there is an interest in finding molecules that inhibit the apoptosome-mediated activation apoptosis.

### DESCRIPTION OF THE INVENTION

One aspect of the present invention relates to the provision of new compounds of general formula 1, 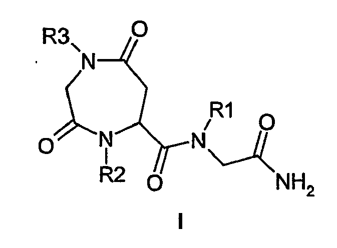 its stereoisomers and mixtures thereof, its polymorphs and mixtures thereof and the pharmaceutically acceptable solvates and addition salts thereof,
wherein:
- R1: represents (C₁-C₅)alkyl, (C₂C₅)alkenyl, -(CH₂)₁₋₃-NHCO-(C₁-C₃)alkyl, -(CH₂)₁₋₃-CONRaRb, -(CH₂)₀₋₃-(3-6)Cy, -(CH₂)₀₋₃-(5-6)Hetcy, -(CH₂)₁₋₃-phenyl, -(CH₂)₁₋₃-(1-naphthyl), -(CH₂)₁₋₃-(2-naphthyl), -(CH₂)₁₋₃-(5-10)Hetar, -(CH₂)₂-CH(phenyl)₂, -(CH₂)₂-CH(phenyl)[(5-6)Hetar] or -(CH₂)₂-CH[(5-6)Hetar]₂;
- R2: represents -(CH₂)₀₋₃-(3-6)Cy, -(CH₂)₀₋₃-(5-6)Hetcy, -(CH₂)₁-₃-phenyl, -(CH₂)₁₋₃-(1-naphthyl), -(CH₂)₁₋₃-(2-naphthyl), -(CH₂)₁₋₃-(5-10)Hetar, -(CH₂)₂-CH(phenyl)₂, -(CH₂)₂-CH(phenyl)[(5-6)Hetar] or -(CH₂)₂-CH[(5-6)Hetar]₂;
- R3: represents -(CH₂)₁₋₃-phenyl, -(CH₂)₁₋₃-(1-naphthyl), -(CH₂)₁₋₃-(2-naphthyl), or -(CH₂)₁₋₃-(5-6)Hetar;
(C₁₋C₅)alkyl and -(C₂₋C₅)alkenyl may be optionally substituted with one or more substituents selected from -O-(C₁₋C₂)alkyl, -S-(C₁-C₂)alkyl, -N.H₂, -NH-(C₁₋C₂)alkyl and -N[(C₁₋C₂)alkyl]₂;
(C₁₋C₃)alkyl can be optionally substituted with one or more halogen atoms;
Ra and Rb independently represent -H or -(C₁-C₃)alkyl
(3-6)Cy represents a radical of a 3- to 6-membered partially unsaturated or saturated carbocyclic ring;
(5-6)Hetcy represents a C- or N- radical of a 5- or 6-membered, partially unsaturated or saturated carbocyclic ring containing from one or two heteroatoms independently selected from O S and N;
Cy and Hetcy can be optionally substituted with one or more substituents selected from halogen, -CF₃ and -OH;
(5-6)Hetar and (5-10)Hetar represent a C- or N- radical of an aromatic 5- or 6-membered and 5- to 10-membered ring respectively; containing from one to four heteroatoms independently selected from O S and N; phenyl, 1-naphthyl, 2-naphthyl, (5-6)Hetar and (5-10)Hetar may be optionally substituted with one or more substituents selected from halogen, -CF3, -OH, -O-(C₁-C₃)alkyl, -CO-(C₁₋C₃)alkyl, -(C₁₋C₅)alkyl-NRaRb, -NRaRb and - SO₂NH₂;
with the proviso that R2 does not represent 2-(4-fluorophenyl)ethyl.

Another aspect of the present invention relates to a compound which is a drug conjugate comprising a radical of formula II 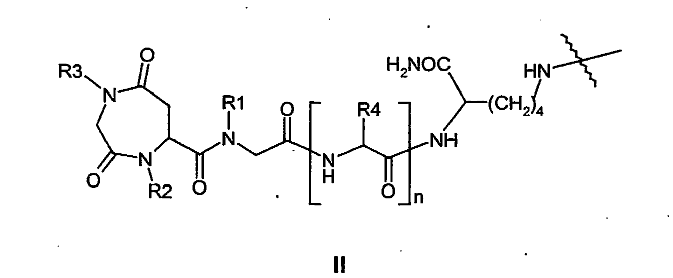 its stereoisomers and mixtures thereof and the pharmaceutically acceptable solvates and addition salts thereof, conjugated to a polyglutamic acid polymer, wherein R1, R2 and R3 have the meanings as defined for the compound of formula I without the R2 proviso; each R4 independently represents a side chain from any of the 20 naturally occurring amino acids, and n is 0,1, 2 or 3.

Thus, compounds of formula II comprise different moieties: a radical of formula I, forming an amide bond to a peptide chain of n L-amino acids and an amide L-lysine derivative, wherein the N of its side chain is linked to the polyglutamic acid polymer.

In the previous definitions, the terms (C₁-C₃)alkyl and (C₁-C₅)alkyl represent straight or branched saturated hydrocarbon chains containing from one to three and from one to five carbon atoms respectively. Examples of (C₁-C₃)alkyl include, but are not limited to, methyl, ethyl, propyl, isopropyl. Examples of (C₁-C₅)alkyl include additionally and without limitation - butyl, isobutyl, sec-butyl, *tert*-butyl, 1-pentyl, 1-methylbutyl, 2-methylbutyl, 3-methylbutyl, 1,1-dimethylpropyl, 1,2-dimethylpropyl and 2,2-dimethylpropyl.

The term (C₂-C₅)alkenyl represents an unsaturated straight or branched hydrocarbon chain, containing from two to five carbon atoms and one or more double bonds, for example ethenyl, 1-propenyl, 2-propenyl, 1-butenyl, 2-butenyl, 3-butenyl and 1,3-butadienyl, 1-pentenyl, 2-pentenyl, 3-pentenyl, 4-pentenyl, 1-methyl-2-butenyl, 2-methyl-3-butenyl, 3-methyl-1-butenyl, 1,1-dimethyl-2-propenyl and 1,2-dimethyl-1-propenyl.

The groups (C₁-C₃)alkyl, (C₁-C₅)alkyl and (C₂-C₅)alkenyl may be optionally substituted according to the description whenever appropriate from a chemical view point.

The term (3-6)Cy represent a radical of a 3- to 6-membered partially unsaturated or saturated carbocyclic ring. As mentioned previously, it can be optionally substituted with one or more substituents, which can be placed at any available position of the ring. Examples of (3-6)Cy include, without limitation, radicals of cyclopropane, cyclobutane, cyclopentane, cyclohexane, 1-cyclobutene, 2-cyclobutene, 1-cyclopentene, 2-cyclopentene, 1-cyclohexene, 2-cyclohexene and 3-cyclohexene.

The term (5-6)Hetcy represents a C- or N- radical radical of a partially unsaturated or saturated 5- to 6-membered carbocyclic ring containing one or two heteroatoms independently selected from O, S and N, that may be substituted according to the description at any available ring position. Examples of (5-6)Hetcy include, without limitation, radicals of dihydrofuran, pyrroline, pyrazoline, imidazolidine, isothiazolidine, isoxazolidine, oxazolidine, pyrazolidine, pyrrolidine, thiazolidine, dioxane, morpholine, piperazine, piperidine, pyran, tetrahydropiran, oxazine, oxazoline, pyrroline, thiazoline, pyrazoline, imidazoline, isoxazoline, and isothiazoline.

The terms (5-6)Hetar and (5-10)Hetar represent a C- or N- radical of an aromatic 5- or 6-membered and 5- to 10-membered ring respectively; containing from one to four heteroatoms independently selected from O, S and N, that may be substituted according to the description at any available ring position. Examples of (5-6)Hetar include, without limitation, radicals of pyrrol, furan, tiophene, imidazole, isoxazole, isothiazole, oxazole, 1,2,4-oxadiazole, 1,2,4-thiadiazole, 1,3,4-oxadiazole, 1,3,4-thiadiazole, 1,2,3-triazole, 1,2,4-triazole, pyridine, pyrimidine, pyridazine and pyrazine. Examples of (5-10)Hetar further include, without limitation, radicals of benzimidazole, benzofuran, benzothiazole, benzothiophene, imidazopyrazine, imidazopyridazine, imidazopyridine, imidazopyrimidine, indazole, indole, isoindole, isoquinoline, pyrazolopyrazine, pyrazolopyridine, pyrazolopyrimidine, purine, quinazoline, quinoline and quinoxaline.

The expression "optionally substituted with one or more" means that a group can be either unsubstituted or substituted with one or more, preferably with 1, 2, 3 or 4 substituents, provided that this group has 1, 2, 3 or 4 positions susceptible of being substituted.

Throughout the description and claims the word "comprise" and variations of the word, such as "comprising", are not intended to exclude other additives, components, elements or steps.

As used therein the term "treatment" includes treatment, prevention and management of such condition. The term "pharmaceutically acceptable" as used herein refers to those compounds, compositions, and/or dosage forms which are, within the scope of medical judgement, suitable for use in contact with the tissues of humans and animals without excessive toxicity, irritation, allergic response, or other problem or complication, commensurate with a reasonable benefit/risk ratio.

In a particular embodiment of the invention R1 represents (C₁-C₅)alkyl, -(CH₂)₁₋₃-CONRaRb, -(CH₂)₀₋₃-(3-6)Cy; -(CH₂)₀₋₃-(5-6)Hetcy; -(CH₂)₁₋₃-(5-10)Hetar, -(CH₂)₁₋₃-phenyl, -(CH₂)₁₋₃-(1-naphthyl) or -(CH₂)₁₋₃-(2-naphthyl), all of them optionally substituted.

In a particular embodiment of the invention R1 represents. -(CH₂)₀₋₃-(5-6)Hetcy, -(CH₂)₁₋₃-(5-10)Hetar, -(CH₂)₁₋₃-_{P}henyl, -(CH₂)₁₋₃-(1-naphthyl) or - CH₂)₁₋₃-(2-naphthyl), all of them optionally substituted.

In another embodiment R1 represents -(CH₂)₂-phenyl optionally substituted with one or more substituents selected from halogen. In another embodiment R1 represents 2,4-dichlorophenyl.

In another embodiment R2 represents -(CH₂)₁₋₃-aryl optionally substituted, or - (CH₂)₂-CH(Ph)₂ optionally substituted, with the proviso that R2 does not represent 2-(4-fluorophenyl)ethyl-. In another embodiment R2 represents 3,3-diphenylpropyl, with the proviso that R2 doers not represent 2-(4-fluorophenyl)ethyl-.

In another embodiment R3 represents -(CH₂)₁₋₃-(5-6)Hetar or -(CH₂)₂-phenyl both optionally substituted. In another embodiment R3 represents -(CH₂)₂- phenyl optionally substituted with one or more substituents selected from halogen. In another embodiment R3 represents 2,4-dichlorophenyl.

In another embodiment R2 represents 3,3-diphenylpropyl and R1 and R3 both represent 2,4-dichlorophenyl.

Another embodiment of the present invention relates to a compound which is a drug conjugate comprising a compound of formula **II** conjugated to a polyglutamic acid polymer as previously defined, wherein said polymer has a molecular weight of from 20 to 60 kDa.
Another embodiment of the invention relates to a compound of formula **III** which is a polymer-drug conjugate 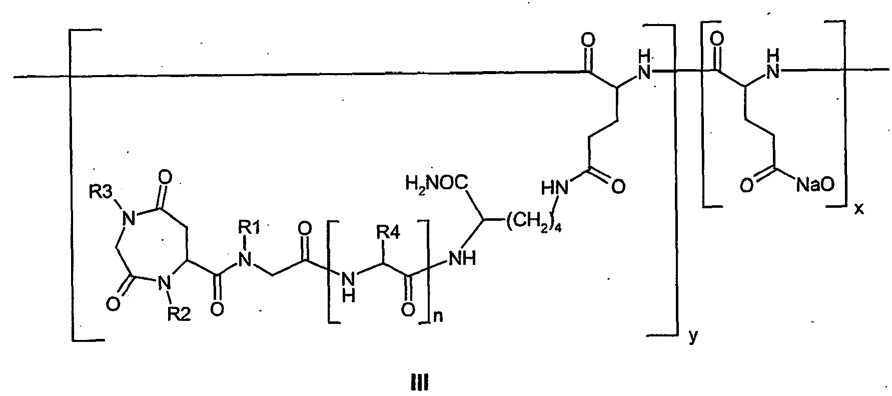 wherein R1, R2, R3 and R4 have the meanings described in the general formula **II** and the proportion of y to x is 25-30% to 75-70% by weight.

In another embodiment in a compound of formula **III** n represents 0. In another embodiment n represents 2 and R4 represents a glycine side chain. In another embodiment n represents 3 and R4 represents a glycine side chain, an arginine side chain and a phenylalanine side chain. In another embodiment n represents 3 and R4 represents a valine side chain, an arginine side chain and a phenylalanine side chain.

In the polymer drug conjugate, the polyglutamic acid may be poly(L-glutamic acid), poly(D-glutamic acid) or poly(DL-glutamic acid). In one embodiment of the invention polyglutamic acid is poly(L-glutamic acid).

Furthermore, all possible combinations of the above-mentioned embodiments form also part of this invention.
Compounds of formula **I** comprise at least one chiral center. The present invention includes both the racemic compounds and the enantiomeric compounds of formula **I**, i.e. compounds wherein the configuration of the chiral carbon attached to R1 is (S) and compounds wherein the configuration of the chiral carbon attached to R1 is (*R*)) as shown below. 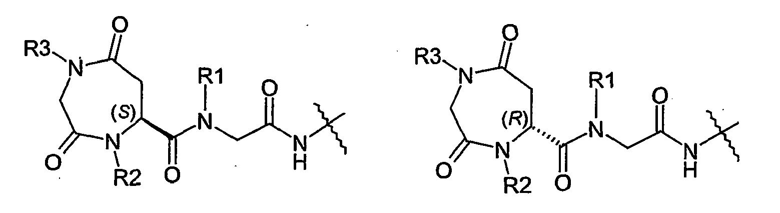
Compounds of formula **II** comprise at least two chiral centers. The present invention also includes both mixtures of stereoisomers and isolated stereoisomers of formula **II.**

The compounds of the present invention may contain one or more basic nitrogen atoms and, therefore, they may form salts with acids, that also form part of this invention. Examples of pharmaceutically acceptable salts include, among others, addition salts with inorganic acids such as hydrochloric, hydrobromic, hydroiodic, nitric, perchloric, sulphuric and phosphoric acid, as well as addition salts of organic acids such as acetic, methanesulfonic, trifluoromethanesulfonic, ethanesulfonic, benzenesulfonic, *p*-toluenesulfonic, benzoic, camphorsulfonic, mandelic, oxalic, succinic, fumaric, tartaric, and maleic acid. Likewise, compounds of the present invention may contain one or more acid protons and, therefore, they may form salts with bases, that also form part of this invention. Examples of these salts include salts with metal cations, such as for example an alkaline metal ion, an alkaline-earth metal ion or an aluminium ion; or it may be coordinated with an organic with an organic or inorganic base. An acceptable organic base includes among others diethylamine and triethylamine. An acceptable inorganic base includes aluminium hydroxide, calcium hydroxide, potassium hydroxide, sodium carbonate, and sodium hydroxide. There may be more than one cation or anion depending on the number of functions with charge and on the valency of cations and anions.

Salts derived from pharmaceutically acceptable organic nontoxic bases include salts of primary, secondary, and tertiary amines, substituted amines including naturally occurring substituted amines, cyclic amines, and basic ion exchange resins, such as arginine, betaine, caffeine, choline, *N,N* dibenzylethylenediamine, diethylamine, 2-diethylaminoethanol, 2-dimethylaminoethanol, ethanolamine, ethylenediamine, *N*-ethylmorpholine, ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like.

There is no limitation on the type of salt that can be used, provided that these are pharmaceutically acceptable when they are used for therapeutic purposes. Salts can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, such as ether, ethyl acetate, ethanol, isopropanol, or acetonitrile or in a mixture of both. The compounds of formula I or formula III and their salts differ in some physical properties but they are equivalent for the purposes of the present invention.

Some of the compounds of formula **I** or formula **III** of the present invention may exist in unsolvated as well as solvated forms such as, for example, hydrates. The present invention encompasses all such above-mentioned forms which are pharmaceutically active.

Some of the compounds of general formula **I** may exhibit polymorphism, encompassing the present invention all the possible polymorphic forms, and mixtures thereof. Various polymorphs may be prepared by crystallization under different conditions or by heating or melting the compound followed by gradual or fast cooling. The presence of polymorphs may be determined by solid NMR spectroscopy, IR spectroscopy, differential scanning calorimetry, powder X-ray diffraction or such other techniques.

Compounds of formula **I** of the present invention comprise at least one chiral center. Additionally, they may have further chiral centres. The present invention includes each one of the possible stereoisomers and mixtures thereof, particularly racemic mixtures thereof. A single enantiomer may be prepared by any of the commonly used processes, for example, by chromatographic separation of the racemic mixture on a stationary chiral phase, by resolution of the racemic mixture by fractional crystallisation techniques of the diastereomeric salts thereof, by chiral synthesis, by enzymatic resolution or by biotransformation. This resolution can be carried out on any chiral synthetic intermediate or on products of general formula I. Alternatively, any enantiomer of a compound of the general formula I may be obtained by enantiospecific synthesis using optically pure starting materials or reagents of known configuration.

Compounds of formula **I** or formula **III** may exist as several diastereoisomers, and may be separated by conventional techniques such as chromatography or fractional crystallization. Some compounds of the present invention may exhibit cis/trans isomers. The present invention includes each of the geometric isomers and its mixtures. The present invention covers all isomers and mixtures thereof (for example racemic mixtures) whether obtained by synthesis and also by physically mixing them.

The present invention relates to a process for the preparation of the above said novel compounds, their tautomeric forms, their stereoisomers, their polymorphs or their pharmaceutical acceptable salts and solvates.

The compounds of the present invention may be synthesized using the methods described below, as well as by other processes known in the field of organic synthesis. Preferred methods include, but are not limited to, the general processes shown in the attached schemes. Unless otherwise stated the groups R1, R2, R3, R4, n, x and y have the meaning described in general formulas **I, II** and **III.**

### Compounds of formula I can be obtained using the following method:

### Method A

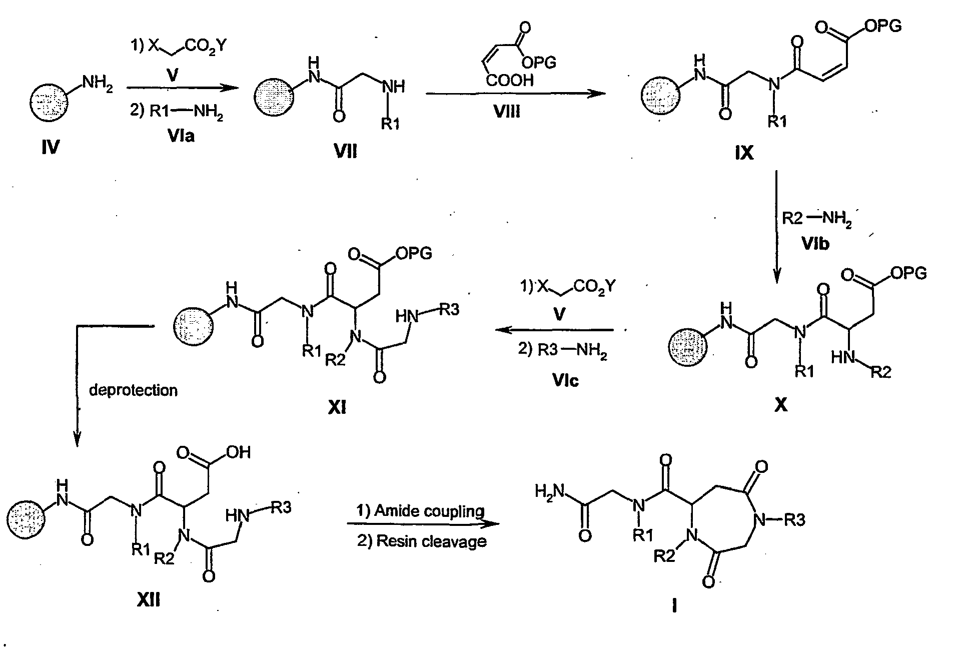

According to Method A, upon deprotection from fluorenylmethoxycarbonyl group, the solid support amine **IV** is acylated with an acylating agent **V,** wherein X represents a leaving group, for example halogen and Y represents - OH or halogen. When Y represents halogen, for example chloroacetylchloride the reaction can be carried out in the presence of a tertiary base as triethylamine. When Y represents -OH, for example bromoacetic acid the reaction can be carried out in the presence of a suitable coupling agent, for example *N,N*'-diisopropylcarbodiimide. In both cases, reaction can be performed in an inert solvent capable of swelling correctly the resin, such as *N,N*'-dimethylformamide or dichloromethane and at room temperature or using microwave irradiation, decreasing reaction time. Then, an amine **VIa** is coupled using a tertiary amine as a base. The reaction can be carried out by heating, for example at 60°C, or by MW irradiation.

A carboxylic acid **VIII,** wherein PG represents a protecting group, such as allyl, is reacted with amine **VII** to yield amide IX using a coupling agent, for example the combination of *N,N*'-diisopropylcarbodiimide and 1-hydroxybenzotriazole. Next, an amine **VIb** is introduced by Michael reaction using a base and a solvent such as *N,N*'-dimethylformamide or dimethylsulfoxide to obtain intermediate **X.** After repetition of acylation and amination in the same way as described for the intermediate **VII, XI** is obtained. Deprotection carried out using PhSiH₃ and Pd(PPh₃)₄ in dichloromethane as a solvent (Tetrahedron Lett 1999, 40:2505-2508; Tetrahedron Lett 1997, 38:7275-7278) or with KOH aqueous in dioxane yields **XII.** Cyclization is promoted by amide formation using standard coupling reagents for solid phase peptidic bond procedures as the combination of benzotriazole-1-yloxy-tris-pyrrolidinophosphonium hexafluorophosphate and 1-hydroxybenzotriazole in presence of a tertiary base as *N,N*-diisopropylethylamine. Final compound **I** can be released from the resin using a mixture of trifluoroacetic acid, dichloromethane and water.

An alternative strategy for obtaining a compound of formula **I** involves acylation of the amines **IV** and **X** with amino acids of formulas **XIIIa** and **XIIIb** respectively (Method B).

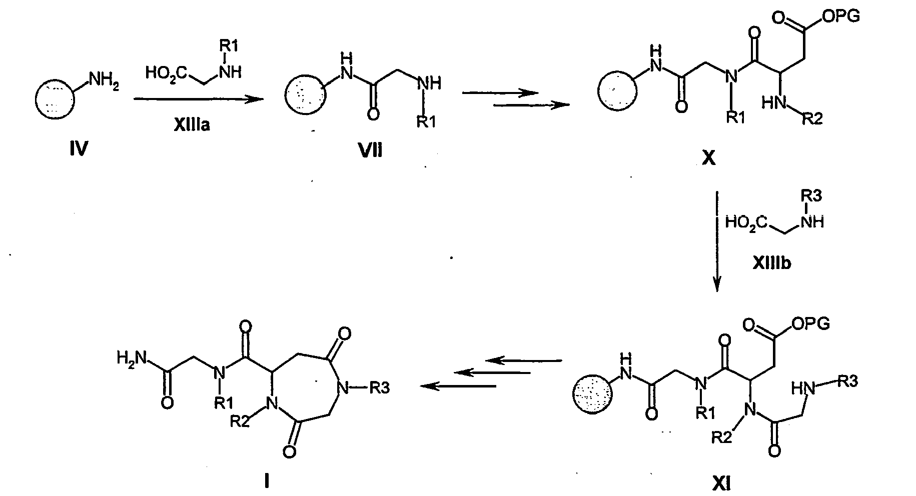

### Method B

As it will be obvious to one skilled in the art, it is possible to combine some steps of method A with some steps of method B to obtain a compound of formula 1.

Starting primary amines **VIa, VIb** and **VIc** are commercially available, or may be obtained by known methods (March, Advanced Organic Chemistry, 1991, Ed. John Wiley & Sons) or using for example the scheme described below.

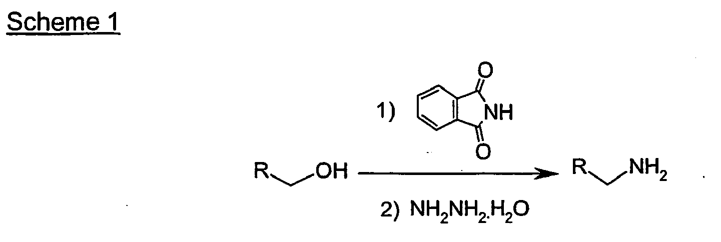

An amine can be obtained by Mitsunobu reaction starting from an alcohol and potassium phthalimide in the presence of, for example, diethyl azodicarboxylate (DEAD) and triphenylphosphine in tetrahydrofurane as a solvent and further cleavage with hydrazine hydrate (Mitsunobu, J. Am. Chem. Soc. 1972, 94, 679-680)

*N*-Substituted glycines **XIIIa** and **XIIIb** may be synthesized by the methods shown below either by reductive amination of the corresponding glycine with the suitable aldehyde (Scheme 2) using reducing agents such as NaBH₄, NaBH₃CN or NaBH(AcO)₃, or by nucleophilic substitution of an ester with the suitable amine E-NH2 (Scheme 3).

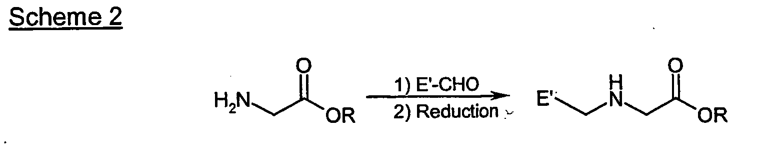

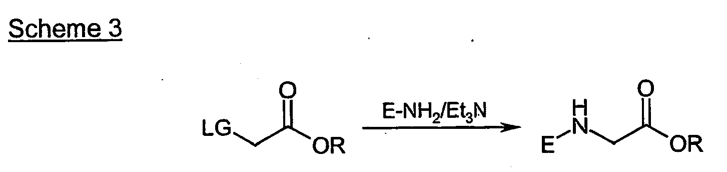

### Compounds of formula II can be obtained using analogous reactions to those decribed above.

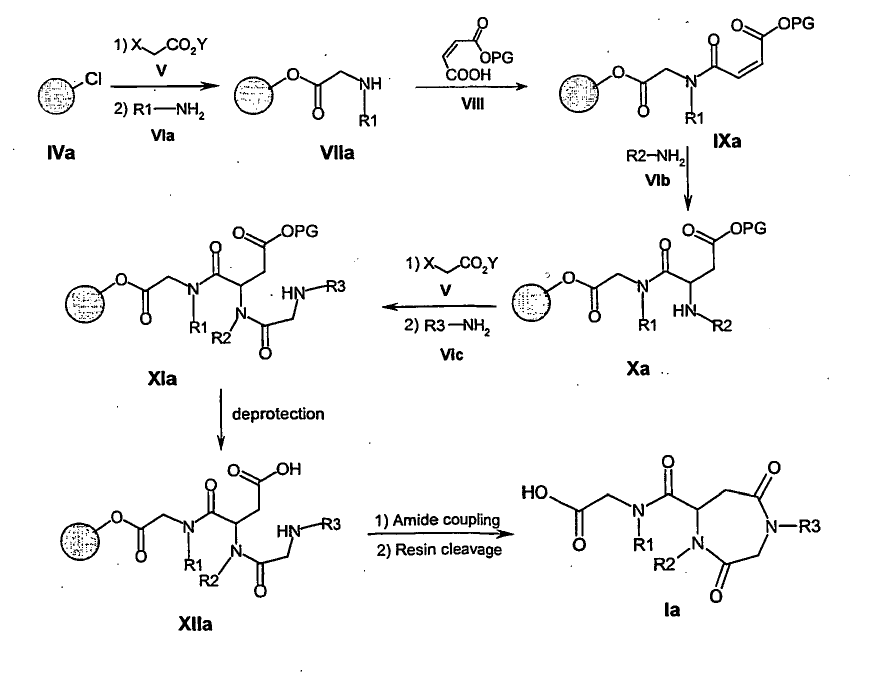

Thus, in order to obtain the corresponding carboxylic acid of formula **I**, that is, a compound of formula **Ia,** the same sequence for the synthesis of a compound of formula **I** is used (using methods A and/or B), except that the starting solid support **(IVa)** has reactive halogen atoms instead of an amino group.

The peptide **XVI** to be bond to compound **Ia** is obtained also using standard reactions for the synthesis of peptides. Thus, the solid support amine **IV** is firstly reacted with a protected Lysine **(XIVa)** and optionally subsequently reacted with another protected amino acid **(XIVb).** After coupling the protecting group is removed. This sequence can be repeated up to three times. 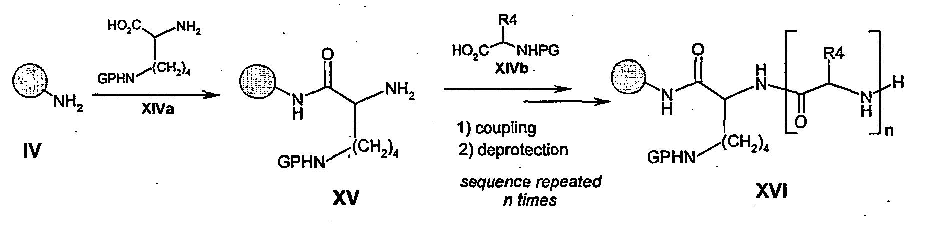 Carboxylic acid **Ia** is then reacted with amine **XVI** and after deprotection compound **IIa** is obtained. Amide coupling between **IIa** and polyglutamic acid **XVIIa** (obtained from the corresponding salt **XVII)** yields the drug conjugate **III.**

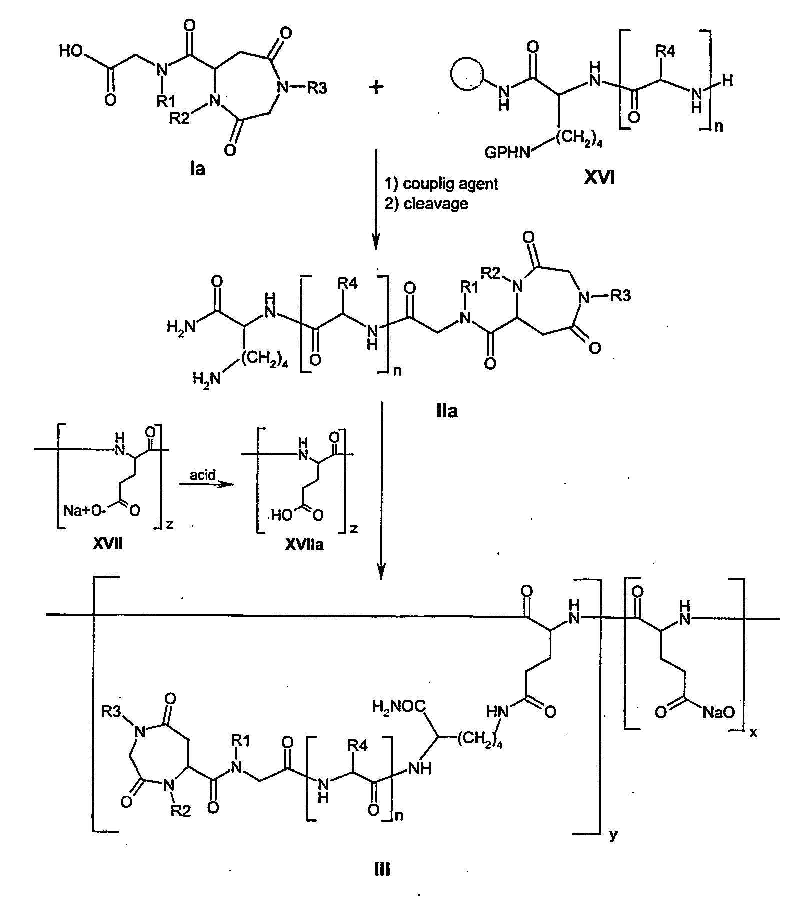

The compounds of the present invention are apaf-1 inhibitors. Therefore, they are useful for the treatment or prevention of a condition associated with the excess of apoptosis.

In adults, post-mitotic cells, such as neurons and heart muscle cells are rarely renewed. Hence their loss can cause neurological and cardiac disorders. These cells undergo apoptotic cell death in a variety of acute and chronic degenerative diseases. Similarly, septic shock, ischaemia, and intoxication can cause massive apoptosis in multiple organs. Infections can cause an elevated apoptotic turnover of specific cell types, for instance, lymphocytes in AIDS or gastric mucosa cells in *Helicobacter pylori* infection. Apoptosis events are also of importance in procedures for ischaemic and preservation-reperfusion in organ transplantation. These diseases are therefore candidates for therapeutic cell death suppression.

Other conditions or diseases related with apoptosis include, without limitation, stroke, traumatisms, brain injury, spinal cord injury, meningitis, Alzheimer's disease, Parkinson's disease, Huntington's disease, Kennedy's disease, multiple sclerosis, prion-related Diseases, as well as other acute or cronic cardiovascular diseases, alcoholic related pathologies, and treatment of allopecia.

Thus, the present invention relates to the use of these compounds for the preparation of a medicament for the treatment or prevention of the above mentioned diseases or conditions.

Another aspect of the present invention relates to the use of a compound of formula I without the R2 proviso or a compound which is a polymer-drug conjugate as previously defined for the manufacture of a medicament for the treatment or prevention of a disease or condition associated with apoptosis.

Described is also a method for the treatment or prevention of a condition associated with the inhibition of apoptosis comprising administering a compound of formula I without the R2 proviso or a compound which is a polymer-drug conjugate as previously defined and one or more pharmaceutical acceptable excipients.

Described is also a method for the treatment or prevention of stroke, traumatisms, brain injury, spinal cord injury, meningitis, Alzheimer's disease, Parkinson's disease, Huntington's disease, Kennedy's disease, multiple sclerosis, prion-related Diseases, myocardial ischaemia, as well as other acute or chronic cardiovascular diseases, organ transplantation, alcoholic related pathologies, and treatment of allopecia, comprising administering a compound of formula **I** without the R2 proviso or a compound which is a polymer-drug-conjugate as previously defined and one or more pharmaceutical acceptable excipients.

The present invention further provides for pharmaceutical compositions comprising a compound of formula **I** or a compound which is a polymer-drug conjugate as previously defined, a pharmaceutically acceptable salt or solvate thereof, together with one or more pharmaceutically acceptable excipients, in either single or multiple doses. The examples of the excipients mentioned below are given by way of illustration only and are not to be construed as limiting the scope of the invention.

The compounds of the present invention can be administered in the form of any pharmaceutical formulation. The pharmaceutical formulation will depend upon the nature of the active compound and its route of administration. Any route of administration may be used, for example such as oral, buccal, pulmonary, topical, parenteral (including subcutaneous, intramuscular, and intravenous), transdermal, ocular (ophthalmic), inhalation, intranasal, otic, transmucosal, implant or rectal administration. However oral, topical or parenteral administration are preferred.

Solid compositions for oral administration include among others tablets, granulates and hard gelatin capsules, formulated both as immediate release or modified release formulations.

Alternatively, the compounds of the present invention may be incorporated into oral liquid preparations such as emulsions, solutions, dispersions, suspensions, syrups, elixirs or in the form of soft gelatin capsules. They may contain commonly-used inert diluents, such as purified water, ethanol, sorbitol, glycerol, polyethylene glycols (macrogols) and propylene glycol. Aid compositions can also contain coadjuvants such as wetting, suspending, sweetening, flavouring agents, preservatives, buffers, chelating agents and antioxidants.

Injectable preparations for parenteral administration comprise sterile solutions, suspensions or emulsions in oily or aqueous vehicles, and may contain coadjuvants, such as suspending, stabilizing, tonicity agents or dispersing agents.

The compound can also be formulated for its topical application. Formulations includes creams, lotions, gels, powders, solutions, shampoo preparations, oral paste, mouth wash preparations and patches wherein the compound is dispersed or dissolved in suitable excipients.

In one embodiment of the invention the pharmaceutical composition is in the form of nanospheres, microparticles and nanoparticles.

The effective dosage of active ingredient may vary depending on the particular compound administered, the route of administration, the nature and severity of the disease to be treated, as well as the age, the general condition and body weight of the patient, among other factors. A representative example of a suitable dosage range is from about 0.001 to about 100 mg/Kg body weight per day, which can be administered as a single or divided doses. However, the dosage administered will be generally left to the discretion of the physician.

### Toxicity and cell recovery through the MTT assay

The MTT assay measures cell proliferation as well as reduction in cell viability when metabolic events lead to apoptosis or necrosis. It can also be used to assess the rate of survival of a given cell line when it is incubated in the presence of xenobiotics. The reduction of tetrazolium salts is accepted as a reliable way to analyse cell proliferation. The yellow tetrazolium MTT (3-(4,5-dimethylthiazolyl-2)-2,5-diphenyltetrazolium bromide) is reduced by metabolically active cells and results in the intracellular formation of purple formazan that can be spectrophotometrically measured after solubilisation with DMSO.
The MTT assay was used in this invention to assess the toxicity of compounds at different concentrations. To analyse their potential impairment in cell viability, compounds were added to Saos-2 and U937 cell lines at different concentrations and incubated at different times. Results are expressed by percentage cell viability.

| | | **% Cell viability** | | | | | |
|---|---|---|---|---|---|---|---|
| | | **24 h, µM.** | | | **48 h, µM.** | | |
| | | **10** | **5** | 1 | **10** | **5** | **1** |
| **Saos-2** | **I.1** | 96 | 100 | 99 | 100 | 100 | 100 |
| **cell line** | **I.10** | 91 | 100 | 76 | 100 | 100 | 100 |
| | **I.2** | 99 | 99 | 100 | 91 | 100 | 100 |
| | **I.11** | 82 | 98 | 100 | 74 | 91 | 100 |
| | **I.4** | 100 | 97 | 100 | 99 | 98 | 100 |
| | **I.9** | 82 | 96 | 100 | 66 | 76 | 97 |
| | **1.3** | 100 | 93 | 97 | 100 | 100 | 100 |
| | **1.7** | 98 | 99 | 95 | 100 | 100 | 100 |
| | **I.8** | 99 | 100 | 100 | 100 | 100 | 97 |
| **U937** | **I.1** | 95 | 98 | 100 | 99 | 97 | 100 |
| **cell line** | **I.10** | 89 | 99 | 96 | 99 | 98 | 100 |
| | **1.2** | 97 | 100 | 100 | 83 | 99 | 100 |
| | **I.11** | 79. | 95 | 100 | 75 | 89 | 98 |
| | **1.4** | 87 | 99 | 100 | 95 | 94 | 99 |
| | **1.9** | 88 | 98 | 100 | 59 | 69 | 93 |
| | **I.3** | 95 | 95 | 100 | 98 | 100 | 100 |
| | **1.7** | 93 | 95 | 99 | 97 | 100 | 100 |
| | **1.8** | 99 | 98 | 100 | 99 | 98 | 100 |

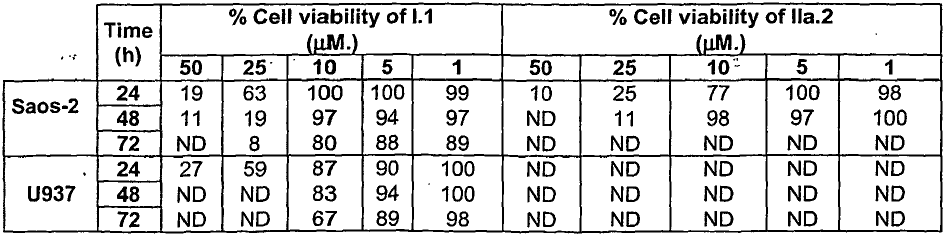

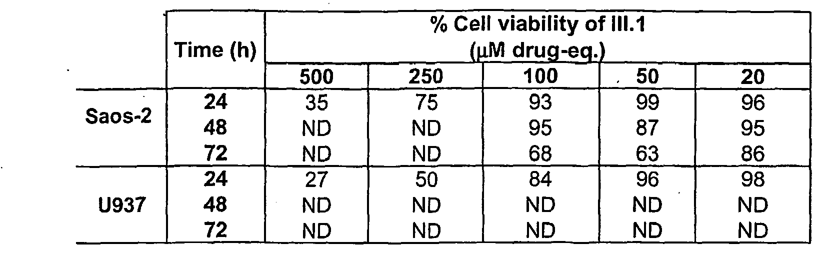

The MTT assay was also used to assess cell recovery by the addition of compounds in apoptosis-induced cell lines. For Saos-2 cell line, apoptosis induction was carried out by the addition of doxycyclin 2 µM; in the case of U937, HeLa, U2-OS, apoptosis was induced by doxorubicin 0.25 µM, 1.5 µM, 2 µM, respectively. Results are expressed as the % of cell viability considering that the addition of doxycyclin and doxorubicin induces 100 % of cell death and 0 % of cell recovery in Saos-2 and HeLa, U2-OS or U937, respectively.

| **Cell Lines** | **% cell recovery** I.1 | | | |
|---|---|---|---|---|
| | **Time (h)** | **10 µm** | **5 µM** | 1 **µm** |
| | **24** | 30 | 31 | 29 |
| **HeLa** | **48** | 3 | 78 | 75 |
| | **72** | 2 | 33 | 34 |
| | **24** | 72 | 0 | 0 |
| **U2-OS** | **48** | 18 | 54 | 58 |
| | **72** | 3 | 25 | 1 |
| | **24** | 52 | 19 | 0 |
| **U937** | **48** | 3 | 19 | 14 |
| | **72** | 4 | 46 | 12 |
| | **24** | 15 | 6 | 76 |
| **Saos-2** | **48** | 8 | 25 | 23 |
| | **72** | 2 | 1 | 3 |

| **Cell Lines** | **% Cell recovery** | | | | | | | | | | | | | | | | |
|---|---|---|---|---|---|---|---|---|---|---|---|---|---|---|---|---|---|
| | | **1.10** | | **I.2** | | **I.11** | | **I.9** | | **I4** | | **I.3** | | **I.7** | | **1.8** | |
| | Time (h) | **5 µm** | **1 µM** | **5 µM** | **1 µM** | **5 µM** | **1 µM** | 5 **µM** | **1 µM** | **5 µM** | **1 µM** | **5 µm** | **1 µM** | **5 µM** | **1 µM** | **5 µM** | **1 µM** |
| **Saos-2** | 24 | 57 | 0 | 10 | 41 | 0 | 22 | 16 | 36 | 9 | 34 | 12 | 18 | 41 | 64 | 23 | 57 |
| | 48 | 24 | 0 | 21 | 21 | 8 | 20 | 22 | 31 | 10 | 24 | 21 | 14 | 19 | 27 | 51 | 24 |

| | | | | | | | | | | | | | | | | | |
|---|---|---|---|---|---|---|---|---|---|---|---|---|---|---|---|---|---|
| ND: Not determined | | | | | | | | | | | | | | | | | |

| | | % Cell recovery III.1 | | |
|---|---|---|---|---|
| | | **100µM** | **50µM** | **20µM** |
| Saos-2 | **24** | 25 | 28 | 15 |
| | **48** | 18 | 49 | 13 |
| | **72** | 15 | 31 | 14 |
| U937 | **24** | 9 | 34 | 5 |
| | **48** | 20 | 9 | 1 |
| | **72** | 10 | 15 | 0 |

### Caspase inhibition assay

The time-dependent inhibition of caspase induced by the compounds described in this invention was analysed. Cell lines were grown in the presence or absence of compounds (50 µM) at different times. Caspase activity was assayed using Saos-2 or U937 cell extracts and Ac-DEVD-afc (N-acetyl-Asp-Glu-Val-Asp-afc(7-amino-4-trifluoromethyl coumarin)) as substrate (20 µM). Caspase activity was continously monitored by the liberation of fluorescent afc using a Cytofluor 4000 fluorimeter (excitation 400 nm; emission 508 nm) for 1 hour. The effect of the compounds on the caspase activity was expressed as the percentage of inhibition in the fluorescence signal of treated cells vs the non-treated cells (control).

| **Cell Lines** | **% Caspase inhibition** | | | | | | | | | | |
|---|---|---|---|---|---|---|---|---|---|---|---|
| | | 1.1 | | I.10 | 1.2 | I.11 | 1.9 | I.4 | 1.3 | 1.7 | I.8 |
| | **Time (h)** | **5 µM** | **1 µM** | **5µM** | **5 µM** | 5 **µM** | **5 µM** | **1 µM** | **5 µM** | **5 µM** | **5 µM** |
| **HeLa** | **24** | 31 | 29 | 1 | 1 | 14 | 0 | 48 | 9 | 6 | 5 |
| | **48** | 78 | 75 | 6 | 46 | 0 | 0 | 14 | 21 | 7 | 3 |
| | **72** | 33 | 34 | ND | 59 | 62 | 41 | 35 | 29 | 11 | 6 |
| **U2-OS** | **24** | 0 | 0 | 0 | 0 | 0 | 0 | 0 | 1 | 0 | 0 |
| | **48** | 54 | 58 | 22 | 54 | 31 | 34 | 23 | 29 | 31 | 16 |
| | **72** | 25 | 1 | 64 | 27 | 50 | 61 | 54 | 22 | 33 | 24 |
| **U937** | **24** | 19 | 0 | 0 | 0 | 11 | 3 | 0 | 12 | 10 | 5 |
| | **48** | 19 | 14 | 5 | 3 | 0 | 4 | 12 | 16 | 8 | 15 |
| | **72** | 46 | 12 | 2 | 0 | 4 | 63 | 0 | 1 | 2 | 23 |
| **Saos-2** | **24** | 53 | 21 | 13 | 11 | 45 | 50 | 55 | 29 | 0 | 1 |
| | **48** | 19 | 17 | 4 | 0 | 7 | 2 | 9 | 16 | 24 | 0 |
| | **72** | 4 | 4 | ND | ND | ND | ND | ND | ND | ND | ND |

| | | | | | | | | | | | |
|---|---|---|---|---|---|---|---|---|---|---|---|
| ND: Not determined | | | | | | | | | | | |

| | **Time (h)** | % Caspase Inhibition **IIa.2** (5 µM.) |
|---|---|---|
| | **24** | 41 |
| **Saos-2** | **48** | 23 |
| | **72** | 1 |
| | **24** | 23 |
| **U937** | **48** | 11 |
| | **72** | 5 |

| **Cell lines** | **Time (h)** | **III.1 % Inh. caspase** |
|---|---|---|
| | | **(50 µM drug-eq.)** |
| **Sagos-2** | **24** | 67 |
| | **48** | 72 |
| | **72** | 100 |
| **U937** | **24** | 74 |
| | **48** | 85 |
| | **72** | 100 |

### Inhibition of Apaf-1-induced apoptosis

The activity of the compounds regards their specific target within the apoptotic machinery was assessed. Apaf-1 oligomerisation is a molecular event that leads to the activation of the apoptosome formation, which ends up in the induction of apoptosis. These experiments were performed in cell-free and in cellular assays.

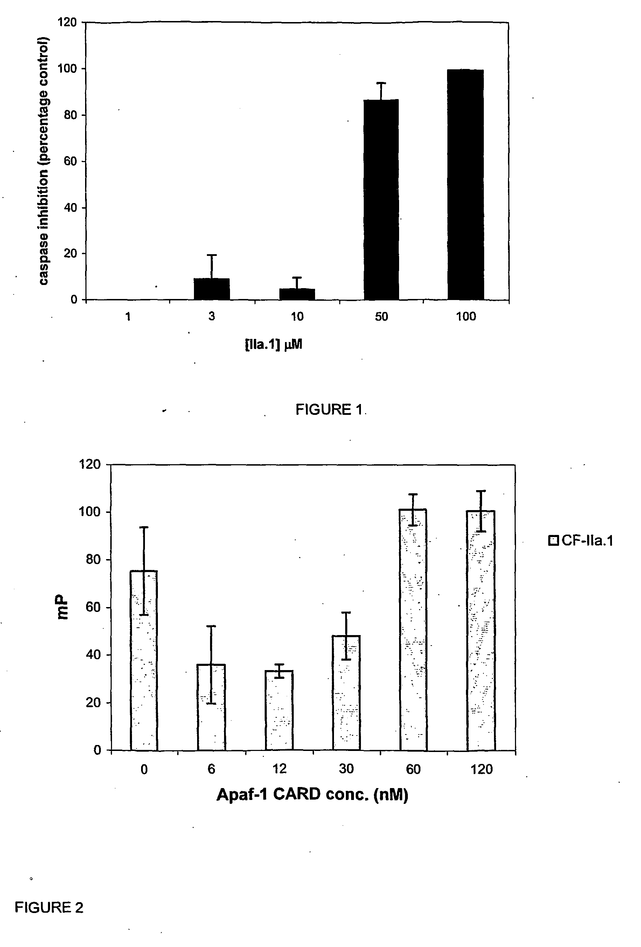

For the cell-free assays, Hek-293 cytoplasmatic extracts were prepared as described before (Malet *et al,* 2005) and a partitioning of the complete cell extract was carried out. Concomitantly, recombinant Apaf-1 was purified according to Malet *et al*, 2005. The FT fraction (containing caspase-3 and -9 but lacking Apaf-1), was incubated with recombinant Apaf-1 in the presence of different concentrations of the compounds (see figure 1). The activity of the compounds was analysed by the inhibition of caspase activity (see above).

For the cellular assays, Saos-2 and U937 cells were treated with an apoptosome activator (Nguyen and Wells, Proc. Natl. Acad. Sci. USA 2003, 100:7533-7538), which specifically induces Apaf-1 oligomerisation. Afterwards, the compounds were added to the cells (50 µM) and incubated for 24 or 48 hours. The capacity of the compounds of inhibiting the Apaf-1 induced apoptosis was analysed by the inhibition of caspase activity (see above).

| **Cell lines** | **Time (h)** | **III.1 % Inh. caspase activity** |
|---|---|---|
| | | **(50 µM drug-eq).** |
| **Saos-2** | **24** | 22 |
| | **48** | 54 |
| **U937** | **24** | 27 |
| | **48** | 95 |

### Binding of IIa.1 to Apaf-1 CARD domain: Fluorescence polarisation assay

Fluorescence polarisation measurements were made in a Victor2V 1420 Multilabel HTS Counter. A 60 nM solution of 5'-6'-carboxyfluoresceinlabelled **IIa.1** (named CF-IIA.1; χexc=480 nm; χem=535 nm) in buffer A (total reaction volume was 200 µl) was titrated with concentrated protein solutions. Data were recorded by using a Wallac 1420 Workstation software. The Kd values were calculate using a two state model (see Figure 2).
To initially characterise the binding site-of the peptoids to the apoptosome, we synthesised a fluorescent analogue of **IIa.1 (CF-IIa.1). CF-IIa.1** bound to Apaf-1 CARD domain, but not to cytochrome c and other control proteins (Malet *et al*, 2005), with dissociation constant (Kd) 60 nM as determined in a fluorescence polarisation assay (Figure 2). Nevertheless, the binding constant value contrasted with the concentration of **IIa.1** that was needed to inhibit 50% of caspase activity in our assays (IC₅₀ = 30 µM, Figure 1). A putative explanation to reconcile these data is that the reticulocyte lysate used to in vitro translate procaspase-9 contained components that diminished the effective concentration of **IIa.1.**

### EXAMPLES

The following abbreviations have been used in the examples:
Boc: *tert-*butoxycarbonyl
DCM: dichloromethane
DIC: *N,N*'-diisopropylcarbodiimide
DIPEA: diisopropylethylamine
DMF: *N,N'*-dimethylformamide
eq: molar equivalent
EtOAc: ethyl acetate
Fmoc: fluorenylmethoxycarbonyl
Glycine: G
HATU: O-(7-azabenzotriazol-1-yl)-*N,N*,*N'N*'-tetramethyluronium hexafluorophosphate
HOBT: 1-hydroxybenzotriazole
Lisine: K
PGA: poly(L-glutamic acid)
Phenylalanine: F
PyBOP: benzotriazole-1-yloxy-tris-pyrrolidinophosphonium
hexafluorophosphate
R: Arginine
rt: retention time
TFA: trifluoroacetic acid
Valine:V

The present invention will be further illustrated by the following examples. The examples are given by way of illustration only and are not to be construed as limiting the scope of the invention.

Polystyrene AM RAM resin (0.75 mmol/g) was purchased from Rapp Polymere GmbH (Germany). 2-Chlorotrityl chloride resin (1.4 mmol/g) was purchased from Novabiochem. Semi-preparative RP-HPLC was performed with a Waters (Milford, MA, U.S.A.) system using a X-Terra C₁₈ (19 x 25 cm, 5 µm) column; eluent: A= 0.1% TFA in water, B = 0.1 % TFA in acetonitrile, gradient:0 min 20% B - 20 min 80%. B.. High resolution mass spectra (HRMS-FAB) were registered at the Mass Spectrometry Service of the University of Santiago de Compostela (Spain).
The nomenclature used in this document is based on AUTONOM (Automatic Nomenclature), a Beilstein Institute computerized system for the generation of IUPAC systematic nomenclature.

### Intermediate VIII

### VIII: (Z)-but-2-enedioc acid monoallyl ester

To a solution of 2 g of maleic anhydride (20 mmol) in chloroform, 1.8 ml of allyl alcohol (26 mmol, 1.3 eq.) were added. The reaction mixture was stirred at 60°C for .50 min under microwave irradiation. The resulting solution was treated with 1 N HCl and extracted with chloroform. The organic extracts were washed with brine, then dried over anhydrous magnesium sulfate and filtered. The solvent was distilled off under reduced pressure, and the obtained residue was purified by column chromatography to obtain intermediate VIII as an oil (purity 95%, yield 85%).

### Compounds of formula I

### I.1: 1-[2-(2,4-Dichlorophenyl)ethyl]-4-(3,3-diphenylpropyl)-3,7-dioxo[1,4]-diazepane-5-carboxylic acid carbamoylmethyl-[2-(2,4-dichlorophenyl)-ethyl]amide

The Rink amide resin (500 mg, 0.4 mmol) was treated with 3 mL of 20% piperidine in DMF and the mixture was stirred in a microwave reactor for 2 min at 60 °C. The resin was filtered and washed with DMF (3 x 3 mL), isopropyl alcohol (3 x 3 mL) and DCM (3 x 3 mL). The resin was treated with a solution of bromoacetic acid (**V**, 275 mg, 5 eq.) and DIC (306 µL, 5 eq.) in DMF (3 mL). The reaction mixture was stirred for 2 min at 60 °C in a microwave reactor. The resin was drained and washed with DCM (3 x 3 mL), isopropyl alcohol (3 x 3 mL) and DMF (3 x 3 mL). A solution of 2,4-dichlorophenethylamine (**VIa**, 300 µL, 5 eq.) and triethylamine (275 µL, 5 eq.) in 3 mL of DMF was added to the resin and the suspension was stirred for 2 min at 90 °C under microwave activation. The supernatant was removed and the residue was drained and washed with DMF (3 x 3 mL), isopropyl alcohol (3 x 3 mL) and DCM (3 x 3 mL). Then, the resin was treated with a solution of (Z)-but-2-enedioic acid monoallyl ester (**VIII**, 310 mg, 5 eq.), HOBT (267 mg, 5 eq.) and DIC (306 µL, 5 eq.) in DCM: DMF (2:1, 3 mL). The reaction mixture was stirred at room temperature for 30 min, filtered and the reaction was repeated under the same conditions. The resin was drained and washed with DCM (3 x 3 mL), isopropyl alcohol (3 x 3 mL) and DMF (3 x 3 mL). Then, a solution of 3,3-diphenylpropylamine (**VIb**, 418 mg, 5 eq.) and triethylamine (275 µL 5 eq.) in 3 mL of DMF was added to the resin and the suspension was stirred for 3 h at room temperature. The reaction was repeated for overnight at the same temperature. The supernatant was removed and the residue was drained and washed with DMF (3 x 3 mL), isopropyl alcohol (3 x3 mL) and DCM (3 x 3 mL). Then, the resin was treated with a solution of bromoacetic acid (**V**, 275 mg, 5 eq.) and DIC (306 µL, 5 eq.) in DMF (3 mL). The reaction mixture was stirred for 2 min at 60 °C in a microwave reactor. The resin was drained and washed with DCM (3 x 3 mL), isopropyl alcohol (3 x 3 mL) and DMF (3 x 3 mL). A solution of 2,4-dichlorophenethylamine (**VIc**, 200 µL, 5 eq.) and triethylamine (210 µL, 5 eq:) in 3 mL of DMF was added to the resin and the suspension was stirred for 2 min at 90 °C under microwave activation. The supernatant was removed, and the residue was drained and washed with DMF (3 x 3 mL), isopropyl alcohol (3 x 3 mL) and DCM (3 x 3 mL). Then, the resin was treated with tetrakis(triphenylphosphine)palladium(0) (46 mg, 0.1 eq.) and phenylsilane (490 µL, 10 eq.) in anhydrous DCM for 15 min at room temperature under an argon atmosphere. This procedure was repeated three times. The supernatant was removed and the residue was drained and washed witch DMF (3 x 3 mL), isopropyl alcohol (3 x 3 mL) and DCM (3 x 3 mL). The cyclization was promoted by treatment with PyBOP (308 mg, 1.5 eq.), HOBT (80 mg, 1.5 eq.) and DIPEA (203 µL, 3 eq.) in DMF (3 mL). The reaction mixture was stirred at room temperature for 16 h and filtered. The resin was drained and washed with DCM (3 x 3 mL), isopropyl alcohol (3 x 3 mL) and DMF (3 x 3 mL). Finally, treatment of the resin with a mixture of 60:40:2 TFA/DCM/water released a crude reaction mixture containing the titled compound **I.1,** which was filtered. Solvents from the filtrate were removed by evaporation under reduced pressure followed by lyophilization. The residue obtained was purified by semipreparative RP-HPLC using aqueous acetonitrile gradient (42% acetonitrilie → 80% acetonitrile, 35 min) to give 91 mg of the desired product (yield 30%, ≥98% purity).
HRMS (M + H)⁺: Calcd. for C₃₉H₃₈Cl₄N₄O₄, 767.1647; found, 767.1720

Following an analogous procedure to that described for example **I1** but using different amines, the following products were obtained:

| **Example** | **Compound name** | **HRMS (M+H)⁺:** | |
|---|---|---|---|
| | | **Calculated** | **found** |
| I.2 | 1-[2-(2,4-Dichlorophenyl)ethyl]-4-(3,3-diphenylpropyl)-3,7-dioxo[1,4]diazepane-5-carboxylic acid carbamoylmethylcyclopropylamide | C₃₄H₃₆Cl₂N₄O₄ 635.2114 | 635.2186 |
| 1.3 | 1-[2-(2,4-Dichlorophenyl)ethyl]-4-(3,3-diphenylpropyl)-3,7-dioxo[1,4]diazepane-5-carboxylic acid (2-acetylaminoethyl)carbamoylmethylamide | C₃₅H₃₉Cl₂N₅O₅ 680.2328 | 680.2401 |
| I4 | 1-[2-(2,4-Dichlorophenyl)ethyl]-4-(3,3-diphenylpropyl)-3,7-dioxo[1,4]diazepane-5-carboxylic acid carbamoylmethyl(tetrahydrofuran-2-ylmethyl)amide | P₃₅H₄₀Cl₂N₄O₅ | 679.2449 |
| I.5 | 1-[2-(2,4-Dichlorophenyl)ethyl]-4-(3,3-diphenylpropyl)-3,7-dioxo[1,4]diazepane-5-carboxylic acid carbamoylmethyl-[2-(4-methoxyphenyl)ethyl]amide | C₄₀H₄₂Cl₂N₄O₅ 729.2532 | 729.2605 |
| I.6 | 1-[2-(2,4-Dichlorophenyl)ethyl]-4-(3,3-diphenyl-propyl)-3,7-dioxo[1,4]diazepane-5-carboxylic acid carbamoylmethylcyclohexylamide | C₃₇H₄₂Cl₂N₄O₄ 677.2583 | 677.2656 |
| I.7 | 1-[2-(2,4-Dichlorophenyl)ethyl]-4-(3,3-diphenylpropyl)-3,7-dioxo[1,4]diazepane-5-carboxylic acid carbamoylmethy-[2-(2-methoxyphenyl)ethyl]amide | C₄₀H₄₂Cl₂N₄O₅ 729.2532 | 729.2605 |
| I.8 | 1-[2-(2,4-Dichlorophenyl)ethyl]-4-(3,3-diphenylpropyl)-3,7-dioxo[1,4]diazepane-5-carboxylic acid carbamoylmethyl[2-(4-chlorophenyl)ethyl]amide | C₃₉H₃₉Cl₃N₄O₄ 733.2037 | 733.2110 |
| I.9 | 1-[2-(2,4-Dichlorophenyl)ethyl]-4-(3,3-diphenylpropyl)-3,7-dioxo[1,4]diazepane-5-carboxylic acid carbamoylmethyl[2-(2-fluorophenyl)-ethyl]amide | C₃₉H₃₉Cl₂FN₄O₄ 717.2332 | 717.2405 |
| I.10 | 1-[2-(2,4-Dichlorophenyl)ethyl]-4-(3,3-diphenylpropyl)-3,7-dioxo[1,4]diazepane-5-carboxylic acid carbamoylmethyl[2-(4-fluorophenyl)ethyl]amide | C₃₉H₃₉Cl₂FN₄O₄ 717.2332 | 717.2405 |
| I.11 | 1-[2-(2,4-Dichloro-phenyl)ethyl]-4-(3,3-diphenyl-propyl)-3,7-dioxo[1,4]diazepane-5-carboxylic acid butyl-carbamoylmethylamide | C₃₅H₄₀Cl₂N₄O₄ 651.2427 | 651.2499 |

### Intermediate Ia

### Ia: {[2-(2,4-Dichlorophenyl)ethyl]-[1-[2-(2,4-dichlorophenyl)ethyl]-4-(3,3-di-phenylpropyl)-3,7-dioxo[1,4]diazepane-5-carbonyl]amino}acetic acid

The 2-chlorotrityl chloride resin (**IVa,** 700 mg, 0.98 mmol) was treated with a solution of chloroacetic acid (**V,** 463 mg, 5 eq.) and DIPEA (840 µL, 5 eq.) in DCM (4 mL). The mixture was stirred at room temperature for 1 h, the reaction was quenched with methanol (550 µL) and stirring was prolonged for 10 min. The supernatant was removed and the residue was drained and washed with DCM (3 x 3mL), isopropyl alcohol (3 x 3 mL) and DMF (3 x 3 mL). A. solution of 2,4-dichlorophenethylamine (**VIa,** 740 µL, 5 eq.) and DIPEA (840 µL, 5 eq.) in 4 mL of DMF was added to the resin and the suspension was stirred for 3 h at room temperature. The supernatant was removed and the residue was drained and washed with DMF (3 x 3 mL), isopropyl alcohol (3 x 3 mL) and DCM (3 x 3 mL). Then, the resin was treated with a solution of (Z)-but-2-enedioic acid monoallyl ester (VIII) (765 mg, 5 eq.), HOBT (662 mg, 5 eq.) and DIC (760 µL, 5 eq.) in 2:1 DCM:DMF (4 mL). The reaction mixture was stirred for 30 min at room temperature, filtered and repeated under the same conditions. Then, a solution of 3,3-diphenylpropylamine (**VIb,** 1.0 g, 5 eq.) and DIPEA (840 µL, 5 eq.) in 4 mL of DMF was added to the resin and the suspension was stirred for 3 h at room temperature. The reaction was repeated stirring was prolonged for 16 h at the same temperature. The supernatant was removed and the residue was drained and washed with DMF (3 x 3 mL), isopropyl alcohol (3 x 3 mL) and DCM (3 x 3 mL). Then, the resin was treated with a solution of chloroacetic acid (V, 463 mg, 5 eq.) and DIC (760 µL, 5 eq.) in 2:1 DCM: DMF (4 mL). The reaction mixture was stirred for 30 min at room temperature. The resin was drained and washed with DCM (3 x 3 mL), isopropyl alcohol (3 x 3 mL) and DMF (3 x 3 mL). A solution of 2,4-dichlorophenethylamine (**VIc**, 740 µL, 5 eq.) and DIPEA (840 µL, 5 eq). in 4 mL of DMF was added to the resin and the suspension was stirred for 3 h at room temperature. The supernatant was removed, and the residue was drained and washed with DMF (3 x 3 mL), isopropyl alcohol (3 x 3 mL) and DCM (3 x 3 mL). Then, the resin was treated with tetrakis(triphenylphosphine)palladium(0) (114 mg, 0.1 eq.) and phenylsilane (1.2 mL, 10 eq.) in anhydrous DCM for 15 min at room temperature under an argon atmosphere. This synthetic step was carried out by triplicate. The supernatant was removed and the residue was drained and washed with DCM (3 x 3 mL), isopropyl alcohol (3 x 3 mL) and DMF (3 x 3 mL). The cyclization was promoted by treatment with PyBOP (765 mg, 1.5 eq.), HOBT (199 mg, 1.5 eq.) and DIPEA (505 µL, 3 eq.) in DMF (4 mL). The mixture was stirred ate room temperature for 48 h and filtered. The resin was drained and washed with DMF (3 x 3 mL), isopropyl alcohol (3x3 mL) and DCM (3 x 3 mL). Finally, treatment of the resin with a mixture of 5:95 TFA/DCM released a crude reaction mixture which was filtered. Solvents from the filtrate were removed under reduced pressure followed by lyophilization to give the compound la (460 mg, yield 61 %, ≥55% purity) as a colourless solid.

### Intermediates IIa

The Rink amide resin (**IV**, 505 mg, 0.4 mmol) was deprotected with 3 mL of 20% piperidine in DMF. The mixture was stirred in a microwave reactor for 2 min at 60 °C. The resin was filtered and washed with DMF (3 x 3 mL), isopropyl alcohol (3 x 3 mL) and DCM (3 x 3 mL). Fmoc-L-Lys(Boc)-OH (**XIVa**, 2 eq) was linked to the resin using HOBT (108 mg, 2 eq.) and DIC (125 µL, 2 eq.). The mixture was stirred for 1 h at room temperature. The resin was drained and washed with DMF (3 x 3 mL), isopropyl alcohol (3 x 3 mL) and DCM (3 x 3 mL). Upon removal of the Fmoc group with 3 mL of 20% piperidine in DMF (30 min), the resin was washed with DMF (3 x 3 mL), isopropyl alcohol (3 x 3 mL) and DCM (3 -x 3 mL). If necessary, the other amino acids (**XlVb**) were coupled under the same conditions as described above to yield intermediate **XVI.**

To the intermediate **XVI** swelled in DMF, acid **Ia** (460 mg, 1.5 eq.) was added in the presence of HATU (228 mg, 1.5 eq.) and DIPEA (205 µL, 3 eq.) in DMF (3 mL). The reaction mixture was stirred at room temperature for 16 h and filtered. The resin was drained and washed with DMF (3 x 3 mL), isopropyl alcohol (3 x 3 mL) and DCM (3 x 3 mL). Treatment of the resin with a mixture of 80:20:2.5:2.5 TFA/DCM/water/ triisopropylsilane afforded released a crude reaction mixture containing compound **IIa,** that was filtered. Solvents from the filtrate were removed under reduced pressure, followed by lyophilization. The residue obtained was purified by semipreparative RP-HPLC using an aqueous-acetonitrile gradient mixture.

| **Example** | **Compound name** | **HPLC-MS** | |
|---|---|---|---|
| | | **rt** | **m/z** |
| IIa.1 (KGG-Ia.1) | 1-[2-(2,4-Dichlorophenyl)ethyl]-4-(3,3-diphenyl-propyl)-3,7-dioxo[1,4]diazepane-5-carboxylic acid {[({[(5-amino-1-carbamoylpentylcarbamoyl)methyl]-carbamoyl}methyl)carbamoyl]methyl}-[2-(2,4-dichlorophenyl)ethyl]amide | 17.0 | 1011,506 |
| IIa.2 (K-Ia.1) | 1-[2-(2,4-Dichlorophenyl)ethyl]-4-(3,3-diphenyl-propyl)-3,7-dioxo[1,4]diazepane-5-carboxylic acid [(5-amino-1-carbamoylpentylcarbamoyl)methyl]-[2-(2,4-dichlorophenyl)ethyl]amide | 17.6 | 895 |
| IIa.3 (KRFG-Ia.1) | 1-[2-(2,4-Dichlorophenyl)ethyl]- 4-(3,3-diphenylpro-pyl)-3,7-dioxo[1,4]diazepane-5-carboxylic acid {[({1-[1-(5-amino-1-carbamoylpentylcarbamoyl)-4-guanidinobutylcarbamoyl]-2-phenylethylcarbamoyl}methyl)carbamoyl]methyl}-[2-(2,4-dichlorophenyl)ethyl]amide | 13.6 | 1257,629 |
| IIa.4 (KRFV-Ia.1) | 1-[2-(2,4-dichlorophenyl)ethyl]-4-(3,3-diphenylpropyl-3,7-dioko[1,4]diazepane-5-carboxylic acid [(1-{1-[1-(5-amino-1-carbamoylpentylcarbamoyl)-4-guanidinobutylcarbamoyl]-2-phenylethylcarbamoyl}-2-methylpropylcarbamoyl)methyl]- 2-(2,4-dichlorophenyl)ethylamide | 17.4 | 1299,650 |

### Compounds of formula III

### III.1: PGA-IIa.1

To a solution of PGA sodium salt (**XVII,** 0.1 g, 0.5 mmol) with a molecular weight (M_{w}) range 17000-4.3000 Da in water, 0.2 M HCI was added until the pH of the solution was adjusted to 2.0. The precipitate was collected, dialyzed against water, and lyophilized. Then PGA in its protinic form (**XVIIa**) was dissolved in dry DMF (7.5 mL) and DIC (3 eq) and HOBt (3 eq) were added and left stirring for 15 min. Then intermediate **IIa.1** (0.052 mmol (12 mol%) 1 eq) was added and the pH adjusted to 8 with DIPEA (1.5 mL). The reaction was allowed to proceed at room temperature for 36 h. Thin layer chromatography (TLC, silica) showed complete conversion of **IIa.1** (R_{f}=0.5) to polymer conjugate (R_{f}=0, MeOH:H₂O:AcOH=85:10:5). To stop the reaction, the mixture was poured into CHCl₃. The resulting precipitate was collected and dried in vacuum. The sodium salt of PGA-peptoid conjugate **III.1** was obtained by dissolving the product in 1.0 M NaHCO₃. This aqueous solution was dialysed against distilled water (M_{w}CO 6,000-8000 Da). Lyophilization of the dialysate yielded the product as a white powder. The total peptoid content in the polymer as determined by UV (λ= 282 nm) was in the range of 25-30 % (w/w) (10-12 mol% functionalization), free drug content < 1wt% total drug.

The average molecular weight (Mw), polydispersity (Mw/Mn) and the behavior of **III.1** and control **XVII** in solution were analyzed by size exclusion chromatography (SEC) and analytical ultracentrifugation (AUC). Both techniques showed III.1 as a more compact conformational structure with smaller hydrodynamic volume and greater sedimentation coefficient (S) than **XVII** (tr = 12.5 min and S= 3.5 ± 0.4 s vs. tr = 11.6 min and S= 1.9 ± 0.1 s for **III.1** and **XVII,** respectively). By sedimentation equilibrium Mw was determined as 26700 Da for **XVII** and 58600 Da for **III.1,** however Mw of 36000 Da (Mw/Mn = 1.8) and 38000 Da (Mw/Mn= 1.5) were determined for **XVII** and **III.1,** respectively, by GPC using PEG standards.

### Determination of total drug content by UV spectroscopy

A stock solution of **IIa** in MeOH was prepared (1 mg/mL). To obtain a calibration curve, samples were diluted using MeOH to give a concentration range of 0-1 mg/mL. The total drug loading of the conjugates was determined by measuring the optical density at 272 nm and 281 nm in H₂O. PGA in the same concentration range as **III** conjugate analyzed (0-5 mg/mL) in H₂O was used as blank. The extinction coefficient found for **IIa** at 281 nm in MeOH at RT was 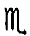 = 713 L mol⁻¹ cm⁻¹.

### Determination of free drug content by HPLC

Aqueous solutions of **III.1** (1 mg/mL) were prepared, and an aliquot (100 µL) was added to a polypropylene tube and made up to 1 mL with water. The pH of the samples was adjusted to 8 with ammonium formate buffer (100 µL, 1 M, pH 8.5), and then CHCl₃ (5 mL) was added. Samples were then thoroughly extracted by vortexing (3 x 10 sec). The upper aqueous layer was carefully removed and the solvent was evaporated under N₂. The dry residue was dissolved in 200 µL of HPLC grade acetonitrile. In parallel the same procedure was carried out for the parent compound **IIa.1** (using 200µL of a 1 mg/mL stock aqueous solution). Addition of 1 mL of acetonitrile to redissolve the product gave a 200 µg/mL stock from which a range of concentrations was prepared (2 to 100 µg/mL). The free amount of drug in the conjugates was determined by HPLC using a Lichrospher^{®}C18 (150 x 3.9 mm) column. Flow rate 1 mL/min using an acetonitrile gradient in aqueous 0.1 % TFA (from 20 to 100% of acetonitrile in 20 min). The retention time was tr = 14.3 min for **IIa.1** (λ = 220 nm).
Following these procedure as for **III.1**, the following examples were obtained:

| **Example** | **Compound name** | **SEC^{a}** | |
|---|---|---|---|
| | | rt | Mw |
| III.1: PGA-IIa.1 conjugate | Poly-L-glutamic acid-[1-[2-(2,4-Dichlorophenyl)ethyl]-4-(3,3-diphenyl-propyl)-3,7-dioxo[1,4]diazepane-5-carboxylic acid {[({[(5-amino-1-carbamoylpentylcarbamoyl)methyl]-carbamoyl}methyl)carbamoyl]methyl}-[2-(2,4-dichlorophenyl)ethyl]amide] conjugate | 12.5 | 36000 |
| III.2: PGA-IIa.2 conjugate | Poly-L-glutamic acid-[1-[2-(2,4-Dichlorophenyl)ethyl]-4-(3,3-diphenyl-propyl)-3,7-dioxo[1,4]diazepane-5-carboxylic acid [(5-amino-1-carbamoylpentylcarbamoyl)methyl]-[2-(2,4-dichlorophenyl)ethyl]amide] conjugate | 13.3 | 34000 |

| | | | |
|---|---|---|---|
| ^{a} Determined by Size Exclusion Chromatography (SEC) | | | |

### DESCRIPTION OF THE DRAWINGS

FIGURE 1: Cell-free caspase inhibition assay using Hek-293 FT cytoplasmatic fraction incubated with recombinant Apaf-1 in the presence of different concentrations the compound **IIa.1**.
FIGURE 2: Analysis of the CARD/**IIa.1** interaction. A 240 nM solution of CF-**IIa.1** in PBS buffer was titrated with increasing concentrations of CARD-Apaf and fluorescence polarization was measured at 30 °C. The data (mean s.d.; n=3) were recorded in millipolarization units (mP).

## Claims

1. - A compound of general formula **I**, 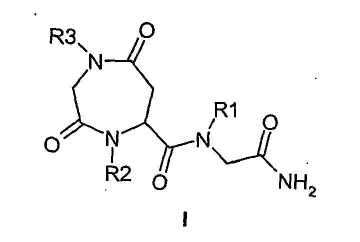 its stereoisomers and mixtures thereof, its polymorphs and mixtures thereof and the pharmaceutically acceptable solvates and addition salts thereof, wherein:
R1 represents (C₁-C₅)alkyl, (C₂-C₅)alkenyl, -(CH₂)₁₋₃-NHCO-(C₁-C₃)alkyl, -(CH₂)₁-₃-CONRaRb, -(CH₂)₀₋₃-(3-6)Cy, -(CH₂)₀₋₃-(5-6)Hetcy, -(CH₂)₁₋₃-phenyl, -(CH₂)₁₋₃-(1-naphthyl), -(CH₂)₁₋₃-(2-naphthyl), -(CH₂)₁₋₃-(5-10)Hetar, -(CH₂)₂-CH(phenyl)₂, -(CH₂)₂-CH(phenyl)[(5-6)Hetar] or -(CH₂)₂-CH[(5-6)Hetar]₂;
R2 represents -(CH₂)₀₋₃-(3-6)Cy, -(CH₂)₀₋₃-(5-6)Hetcy, -(CH₂)₁₋₃-phenyl, -(CH₂)₁₋₃-(1-naphthyl), -(CH₂)₁₋₃-(2-naphthyl), -(CH₂)₁₋₃-(5-10)Hetar, -(CH₂)₂-CH(phenyl)₂, -(CH₂)₂-CH(phenyl)[(5-6)Hetar] or -(CH₂)₂-CH[(5-6)Hetar]₂;
R3 represents -(CH₂)₁₋₃-phenyl, -(CH₂)₁₋₃-(1-naphthyl), -(CH₂)₁₋₃-(2-naphthyl), or -(CH₂)₁₋₃-(5-6)Hetar;
(C₁-C₅)alkyl and -(C₂-C₅)alkenyl may be optionally substituted with one or more substituents selected from -O-(C₁-C₂)alKyl, -S-(C₁-C₂)alkyl, -NH₂, -NH-(C₁-C₂)alkyl and -N[(C₁-C₂)alkyl]₂;
(C₁-C₃)alkyl can be optionally substituted with one or more halogen atoms;
Ra and Rb independently represent -H or -(C₁-C₃)alkyl
(3-6)Cy represents a radical of a 3- to 6-membered partially unsaturated or saturated carbocyclic ring;
(5-6)Hetcy represents a C- or N- radical of a 5- or 6-membered, partially unsaturated or saturated carbocyclic ring containing from one or two heteroatoms independently selected from O, S and N;
Cy and Hetcy can be optionally substituted with one or more substituents selected from halogen, -CF₃ and -OH;
(5-6)Hetar and (5-10)Hetar represent a C- or N- radical of an aromatic 5- or 6-membered and 5- to 10-membered ring respectively; containing from one to four heteroatoms independently selected from O, S and N;
phenyl, 1-naphthyl, 2-naphthyl, (5-6)Hetar and (5-10)Hetar may be optionally substituted with one or more substituents selected from halogen, -CF₃, -OH, -O-(C₁-C₃)alkyl, -CO-(C₁-C₃)a)ky), -(C₁-C₅)alkyl-NRaRb, -NRaRb and -SO₂NH₂;
with the proviso that R2 does not represent 2-(4-fluorophenyl)ethyl.

2. A compound according to claim 1 wherein R1 represents -(CH₂)₀₋₃-(5-6)Hetcy, -(CH₂)₁₋₃-(5-10)Hetar, -(CH₂)₁₋₃-phenyl, -(CH₂)₁₋₃-(1-naphthyl) or -(CH₂)₁₋₃-(2-naphthyl), all of them optionally susbtituted.

3. A compound according to claim 1 or 2 wherein R2 represents -(CH₂)₁-₃-phenyl, -(CH₂)₁₋₃-(1-naphthyl), -(CH₂)₁₋₃-(2-naphthyl) or -(CH₂)₂-CH(phenyl)₂, all of them optionally substituted, with the proviso that R2 does not represent 2-(4-fluorophenyl)ethyl.

4. A compound according to any of claims 1 to 3, wherein R3 represents - (CH₂)₁-₃-(5-6)Hetbr or -(CH₂)₂-phenyl, both optionally substituted.

5. - A compound which is a drug conjugate comprising a radical of formula II 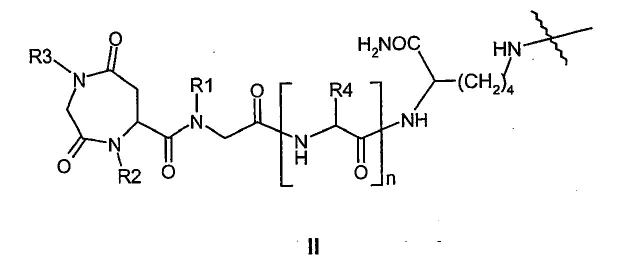 its stereoisomers and mixtures thereof and the pharmaceutically acceptable solvates and addition salts thereof, conjugated to a polyglutamic acid polymer, wherein R1, R2 and R3 have the meanings as defined in claim 1 without the R2 proviso; each R4 independently represents a side chain from any of the 20 naturally occurring amino acids, and n is 0,1, 2 or 3.

6. - A compound of formula III which is a drug conjugate according to claim 2 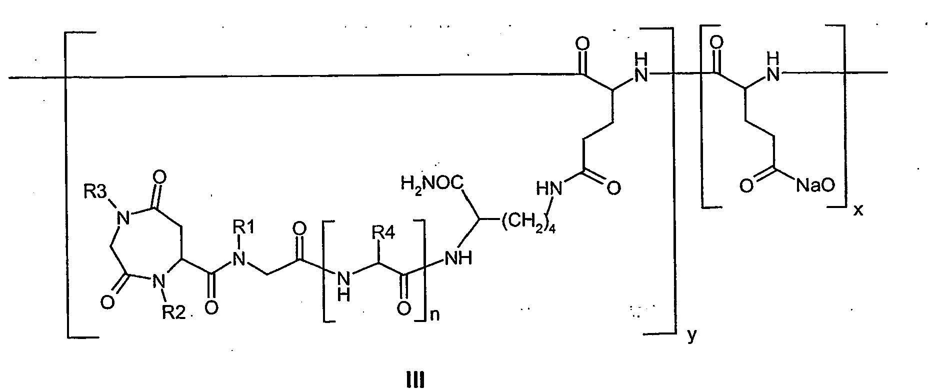 wherein the proportion of y to x is 25-30% to 75-70% by weight.

7. - A compound according to claim 5 or 6 wherein the polyglutamic acid is poly(L-glutamic acid); n represents 2 and R4 represents a glycine side chain.

8. - Use of a compound of formula **I** as defined in claim 1 without the R2 proviso or a compound as defined in claim 5 for the manufacture of a medicament for the treatment or prevention of a condition associated with the inhibition of apoptosis.

9. - Use according to claim 8 wherein the condition is selected from stroke, traumatisms, brain injury, spinal cord injury, meningitis, Alzheimer's disease, Parkinson's disease, Huntington's disease, Kennedy's disease, multiple sclerosis, prion-related Diseases, miocardial ischaemia, as well as other acute or chronic cardiovascular diseases, organ transplantation, alcoholic related pathologies or treatment of allopecia.

10. - A pharmaceutical composition which comprises an effective amount of a compound of formula I as defined in claim 1 or a compound as defined in claim 5, a pharmaceutically acceptable salt or solvate thereof, and one or more pharmaceutical acceptable excipients.

11. - A pharmaceutical composition according to claim 12 in the form of nanospheres, microparticles and nanoparticles.

## Patentansprüche

1. Verbindung der allgemeinen Formel I, 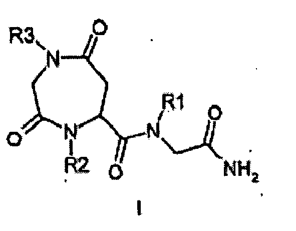 ihre Stereoisomere und Mischungen derselben, ihre Polymorphe und Mischungen derselben und pharmazeutisch akzeptable Solvate und Additionssalze derselben, wobei:
R1 (C₁-C₅) -Alkyl, (C₂-C₅) -Alkenyl, -(CH₂)₁₋₃-NHCO-(C₁-C₃)-Alkyl, -(CH₂)₁₋₃-CONRaRb, -(CH₂)₀₋₃-(3-6) Cy,-(CH₂)₀₋₃-(5-6)Hetcy, -(CH₂)₁₋₃-Phenyl, -(CH₂)₁₋₃(1-Naphthyl), -(CH₂)₁₋₃-(2-Naphthyl), -(CH₂)₁₋₃-(5-10) Hetar, -(CH₂)₂-CH(Phenyl)₂, -(CH₂)₂-CH(Phenyl)[(5-6)Hetar] oder - (CHz)2-[(5-6) Hetar]2 darstellt;
R2 -(CH₂)₀₋₃-(3-6)Cy, -(CH₂)₀₋₃-(5-6)Hetcy, -(CH₂)₁₋₃-Phenyl, -(CH₂)₁₋₃-(1-Naphthyl), -(CH₂)₁₋₃- (2-Naphthyl), -(CH₂)₁₋₃-(5-10) Hetar, - (CH₂)₂-CH(Phenyl)₂, -(CH₂)₂-CH(Phenyl)[(5-6)Hetar] oder-(CH₂)₂-[(5-6)Hetar]₂ darstellt,;
R3 -(CH₂)₁₋₃-Phenyl, -(CH₂)₁₋₃-(1-Naphthyl), -(CH₂)₁₋₃-(2-Naphthyl) oder - (CH₂)₁₋₃-(5-10) Hetar darstellt;
(C₁-C₅)-Alkyl und -(C₂-C₅)-Alkenyl wahlweise durch einen oder mehrere Substituenten substituiert sein können, ausgewählt aus der Gruppe bestehend aus -O- (C₁-C₂)-Alkyl, -S-(C₁-C₂)-Alkyl, -NH₂, -NH-(C₁-C₂)-Alkyl und -N[C₁-C₂)-Alkyl]₂;
(C₁-C₃) -Alkyl wahlweise durch ein oder mehrere Halogenatome substituiert sein kann;
Ra und Rb unabhängig -H oder -(C₁-C₃)-Alkyl darstellen;
(3-6)Cy ein Radikal eines 3- bis 6-gliedrigen, teilweise ungesättigten oder gesättigten carbocyclischen Rings darstellt;
(5-6)Hetcy ein C- oder N-Radikal eines 5- oder 6-gliedrigen, teilweise ungesättigten oder gesättigten carbocyclischen Rings darstellt, der ein bis zwei Heteroatome enthält, die unabhängig unter O, S und N ausgewählt sind;
Cy und Hetcy wahlweise durch einen oder mehrere Substituenten substituiert sein können, ausgewählt unter Halogen, -CF₃ und -OH;
(5-6₋)Hetar und (5-10)Hetar ein C- oder N-Radikal eines aromatischen 5- oder 6-gliedrigen bzw. 5- bis 10-gliedrigen Rings darstellen, die ein bis vier Heteroatome aufweisen, die unabhängig unter O, S und N ausgewählt sind;
Phenyl, 1-Naphthyl, 2-Naphtyl, (5-6)Hetar und (5-10)Hetar wahlweise durch einen oder mehrere Substituenten substituiert sein können, ausgewählt unter Halogen, -CF₃, -OH, O-(C₁-C₃)-Alkyl, -CO- (C₁-C₃)-Alkyl, - (C₁-C₅) -Alkyl-NRaRb, -NRaRb und -SO₂NH₂;
mit der Maßgabe, dass R2 nicht 2-(4-Fluorphenyl)ethyl darstellt.

2. Verbindung nach Anspruch 1, wobei R1 -(CH₂)₀₋₃-(5-6) Hetcy, -(CH₂)₁₋₃-(5-10) Hetar, -(CH₂)₁₋₃(Phenyl), (CH₂)₁₋₃-(1-Naphthyl) oder -(CH₂)₁₋₃-(2-Naphthyl) darstellt, wobei alle derselben wahlweise substituiert sind.

3. Verbindung nach Anspruch 1 oder 2, wobei R2 -(CH₂)₁₋₃-Phenyl, -(CH₂)₁₋₃-(1-Naphthyl), -(CH₂)₁₋₃-(2-Naphthyl), oder -(CH₂)₂-CH(Phenyl)₂ darstellt, wobei alle derselben wahlweise substituiert sind, mit der Maßgabe, dass R2 nicht 2-(4-Fluorphenyl)ethyl darstellt.

4. Verbindung nach einem der Ansprüche 1 bis 3, wobei R3 - (CH₂)₁₋₃-(5-6) Hetar oder -(CH₂)₂-Phenyl darstellt, die beide wahlweise substituiert sind.

5. Verbindung, die ein Arzneimittelkonjugat ist, das ein Radikal der Formel II 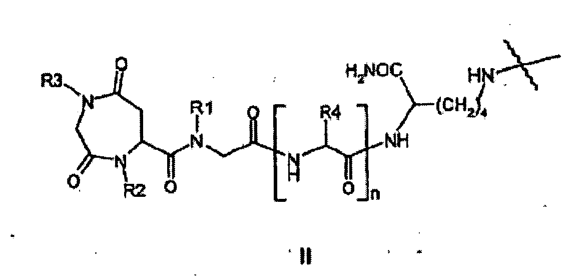 umfasst, ihre Stereoisomere und Mischungen derselben und die pharmazeutisch akzeptablen Solvate und Additionssalze derselben, die an ein Polyglutaminsäurepolymer konjugiert sind, umfasst, wobei R1, R2 und R3 die Bedeutungen wie in Anspruch 1 ohne die Maßgabe für 2 besitzen; jedes R4 unabhängig eine Seitenkette von irgendeiner der 20 natürlich vorkommenden Aminosäuren darstellt und n 0, 1, 2 oder 3 beträgt.

6. Verbindung der Formel III, die ein Arzneimittelkonjugat nach Anspruch 2 ist 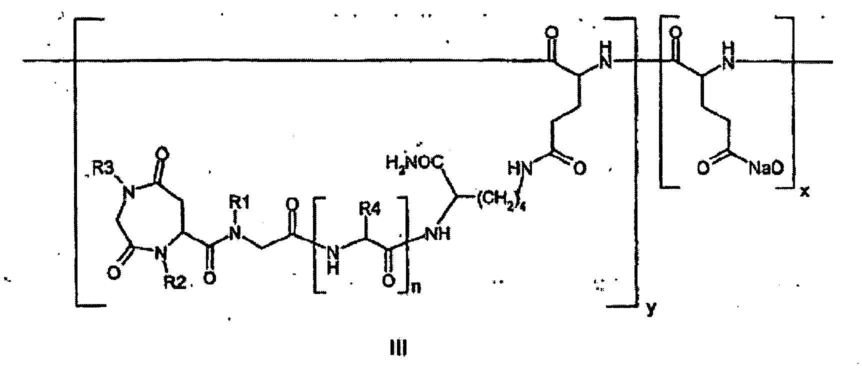 wobei das Verhältnis von y zu x 25-30 Gew.-% bis 75-70 Gew.-% beträgt.

7. Verbindung nach Anspruch 5 oder 6, wobei die Polyglutaminsäure Poly(L-glutaminsäure) ist; n 2 beträgt und R4 eine Glycinseitenkette darstellt.

8. Verwendung einer Verbindung der Formel I wie in Anspruch 1 definiert, ohne die Maßgabe für R2, oder einer Verbindung wie in Anspruch 5 definiert, für die Herstellung eines Medikaments für die Behandlung oder Prävention eines Zustands, der mit der Hemmung von Apoptose verbunden ist.

9. Verwendung nach Anspruch 8, wobei der Zustand unter Schlaganfall, Traumatismus, Gehirnverletzung, Rückenmarkverletzung, Hirnhautentzündung, Alzheimer-Krankheit, Parkinson-Krankheit, Huntington-Chorea, Kennedy-Syndrom, Multipler Sklerose, mit Prionen verbundenen Krankheiten, myokardialer Ischämie sowie anderen akuten oder chronischen kardiovaskulären Krankheiten, Organtransplantation, mit Alkohol verbundenen Pathologien oder Behandlung von Allopezie ausgewählt wird.

10. Pharmazeutische Zusammensetzung, die eine wirksame Menge einer Verbindung der Formel I wie in Anspruch 1 definiert, oder eine Verbindung wie in Anspruch 5 definiert, ein pharmazeutisch akzeptables Salz oder Solvat derselben und ein oder mehrere pharmazeutisch akzeptable Vehikel umfasst.

11. Pharmazeutische Zusammensetzung nach Anspruch 12 in Form von Nanosphären, Mikropartikeln und Nanopartikeln.

## Revendications

1. Composé de formule générale 1, 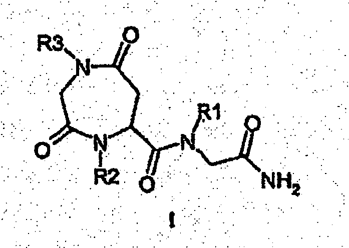 ses stéréo-isomères et mélanges de ceux-ci, ses polymorphes et mélanges de-ceux-ci et ses solvates et sels d'addition pharmaceutiquement acceptables, dans lequel :
R1 représente un alkyle en (C₁-C₅), un alcényle en (C₂-C₅), un -(CH₂)₁₋₃-NHCO- alkyle en (C₁-C₃), -(CH₂)₁₋₃-CONRaRb, -(CH₂)₀-₃-(3-6)Cy, -(CH₂)₀-₃-(5-6)Hetcy, -(CH₂)₁₋₃-phényle, -(CH₂)₁₋₃-CH(phényle)₂, -(CH₂)₂-CH(phényle)([5-6)Hetar] ou -(CH₂)₂-CH[(5-6)Hetar]₂ ;
R2 représente un -(CH₂)₀₋₃-(3-6)Cy, -(CH₂)₀₋₃-(5-6)Hetcy, -(CH₂)₁₋₃-phényle, -(CH₂)₁₋₃-(1-naphtyle),-(CH₂)₁₋₃- (2-naphtyle), -(CH₂)₁₋₃-(5-10) Hetar, -(CH₂)₂-CH(phényle)₂, -(CH₂)₂-CH(phényle)[(5-6)Hetar] ou-(CH₂)₂-CH[(5-6)Hetar]₂ ;
R3 représente un - (CH₂)₁₋₃-phényle, -(CH₂)₁₋₃-(1-naphtyle), -(CH₂)₁₋₃-(2-naphtyle) ou -(CH₂)₁₋₃-(5-6)Hetar ;
l'alkyle en (C₁-C₅) et l'alcényle en (C₂-C₅) peuvent être éventuellement substitués par un ou plusieurs substituant choisis parmi un -O-alkyle en (C₁-C₂), -S-alkyle en (C₁-C₂), -NH₂, -NH-alkyle en (C₁-C₂), et N[(alkyle en (C₁-C₂)]₂ ;
l'alkyle en (C₁-C₃) peut être éventuellement substitué par un ou plusieurs atomes d'halogène ;
Ra et Rb représentent indépendamment un -H ou un-alkyle en (C₁-C₃).
(3-6)Cy représente un radical de cycle carbocyclique saturé ou partiellement insaturé à 3 à 6 éléments ;
(5-6)Hetcy représente un radical C ou N d'un cycle carbocyclique saturé ou partiellement insaturé à 5 ou 6 éléments contenant d'un ou deux hétéroatomes indépendamment choisis parmi O, S et N ;
Cy et Hetcy peuvent être éventuellement substitués par un ou plusieurs substituants choisis parmi un halogène, um -CF₃ et -OH ;
(5-6)Hetar et (5-10)Hetar représentent un radical C- ou N- d'un cycle aromatique à 5 ou 6 éléments et à 5 ou 10 éléments respectivement ; contenant d'un à quatre hétéroatomes indépendamment choisis parmi un O, S et N ;
un phényle, 1-naphtyle, 2-naphtyle, (5-6)Hetar et (5-10)Hetar peuvent être éventuellement substitués par un ou plusieurs substituants choisis parmi un halogène, un -CF₃, -OH, -O-alkyle en (C₁-C₃), -CO-alkyle en (C₁-C₃), -alkyle en (C₁-C₅)-NRaRb, -NRaRb, et -SO₂NH₂ ;
à condition que R2 ne représente pas un 2-(4-fluorophényl)éthyle.

2. Composé selon la revendication 1, dans lequel R1 représente un -(CH₂)₀₋₃-(5-6)Hetcy, - (CH₂)₁₋₃-(5-10)Hetar, (CH₂)₁₋₃-phényle, -(CH₂)₁₋₃-(1-naphtyle) ou-(CH₂)₁₋₃- (2-naphtyle) tous étant éventuellement substitués.

3. Composé selon la revendication 1 ou 2, dans lequel R2 représente un -(CH₂)₁₋₃-phényle, un -(CH₂)₁₋₃-(1-naphtyle), un -(CH₂)₁₋₃-(2-naphtyle) ou (CH₂)₂-CH(phényle)₂, tous étant éventuellement substitués, à condition que R2 ne représente pas un 2-(4-fluorophényl)éthyle.

4. Composé selon l'une quelconque des revendications 1 à 3, dans lequel R3 représente un-(CH₂)₁-₃- (5-6) Hetar ou un -(CH₂)₂-phényle, tous deux éventuellement substitués.

5. Composé qui est un conjugué de médicament comprenant un radical de formule II 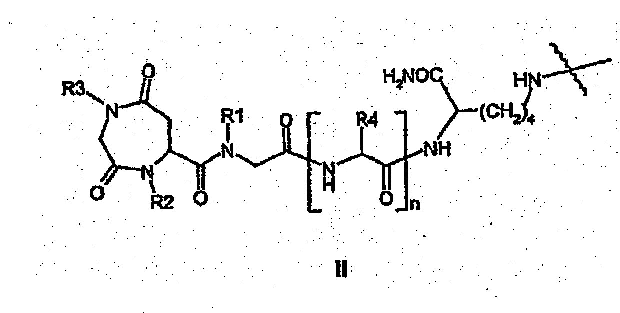 ses stéréo-isomères et mélanges, et ses solvates et sels d'addition pharmaceutiquement acceptables, conjugués à un polymère d'acide polyglutamique, où R1, R2 et R3 ont les significations définies dans la revendication 1, sans la condition de R2 ; chaque R4 représentant indépendamment une chaîne latérale de l'un quelconque des 20 acides aminés naturels, et n est égal à 0, 1, 2 ou 3.

6. Composé de formule III qui est un conjugué de médicament selon la revendication 2, 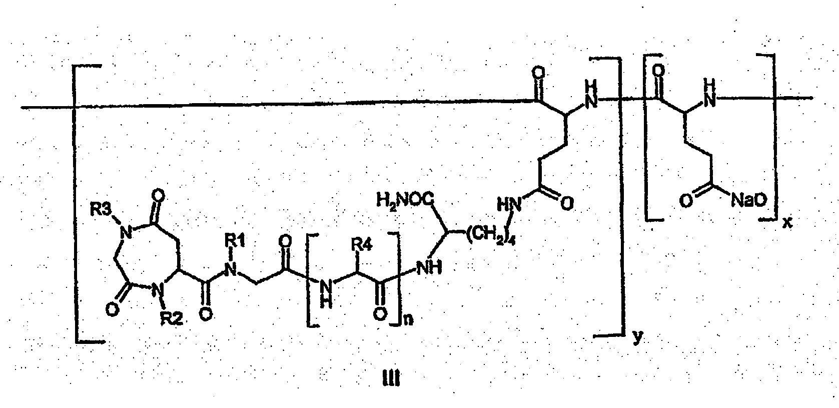 où la proportion de y sur x est de 25-30% à 75-70% en poids.

7. Composé selon la revendication 5 ou 6, dans lequel l'acide polyglutamique est un acide poly(L-glutamique) ; n représente 2 et R4 représente une chaîne latérale de glycine.

8. Utilisation d'un composé de formule I comme défini dans la revendication 1, sans la condition de R2
ou d'un composé selon la revendication 5, pour la fabrication d'un médicament pour le traitement ou la prévention d'une condition associée à l'inhibition de l'apoptose.

9. Utilisation selon la revendication 8, dans laquelle la condition est choisie parmi une attaque, des traumatismes, une lésion cérébrale, une lésion de la moelle épinière, la méningite, la maladie d'Alzheimer, la maladie de Parkinson, la maladie de Huntington, la maladie de Kennedy, la sclérose en plaques, les maladies liées au prison, l'ischémie myocardique, ainsi que d'autres maladies cardiovasculaires aigues ou chroniques, la greffe d'organes, les pathologies liées à l'alcoolisme ou le traitement de l'alopécie.

10. Composition pharmaceutique qui comprend une quantité efficace d'un composé de formule I comme défini dans la revendication 1, ou d'un composé comme défini dans la revendication 5, son sel ou solvate pharmaceutiquement acceptable, et un ou plusieurs excipients pharmaceutiques acceptables.

11. Composition pharmaceutique selon la revendication 10 sous la forme de nanobilles, microparticules et nanoparticules.
